# EUROPEAN PATENT APPLICATION

(11) **EP 1 842 912 A1**
(43) Date of publication of application: **10.10.2007**
(21) Application number: 05814767.9
(22) Date of filing: 12.12.2005
(51) Int. Cl.: C12N 15/09, C12N 1/21, C12P 7/40, C12P 7/52, C12P 7/54, C12P 21/02

(54) **METHOD OF BREEDING CELLS HAVING IMPROVED TOLERANCE TO SHORT-CHAIN FATTY ACIDS**

(30) Priority: 17.12.2004 JP 2004365521; 24.01.2005 JP 2005014984
(71) Applicant: Mitsukan Group Corporation, Handa-shi, Aichi 475-8585 (JP)
(72) Inventor: NAKANO, Shigeru, Chita-gun, Aichi 4702212 (JP); FUKAYA, Masahiro, Chita-gun, Aichi 470-2101 (JP)
(74) Representative: Gille Hrabal Struck Neidlein Prop Roos
(86) International application number: PCT/JP2005/022742
(87) International publication number: WO 2006/064746

(57) **Abstract**

It is intended to provide a method of conferring to microbial cells or the like, tolerance to a short chain fatty acid, including formic acid and acetic acid, which is harmful to the growth thereof without suppressing the growth of the cells while maintaining a high productivity of useful substances. It is also intended to provide a means for efficiently culturing a microorganism in the presence of a short chain fatty acid, or a means for producing a useful short chain fatty acid via fermentation.

The present invention provides a method of breeding cells to improve tolerance to a short chain fatty acid, wherein a gene encoding a protein having a function of transporting a short chain fatty acid from the inside of cells to the outside of cells, is transformed into and expressed in the cells; a high cell-density culture method for producing useful substances using the cell; and a method of preparing a fermentation broth comprising a short chain fatty acid, wherein the cells are cultured under the conditions such that the cells produce a short chain fatty acid.

## Description

### [Technical field]

The present invention relates to a method of breeding cells to improve tolerance to a short chain fatty acid, and in particular, to a method of breeding cells to improve tolerance to a short chain fatty acid by introducing a gene conferring tolerance to a short chain fatty acid into cells of a microorganism or the like, and expressing the gene therein, and as for the use of such microbial cells obtained by the method, to a method for high cell-density culture for producing useful substances, and efficient preparation of a fermentation broth comprising a short chain fatty acid.

### [Background art]

It is conventional to add, in industrial culture of microorganisms, carbon sources such as glucose and the like, nitrogen sources such as peptone and the like, inorganic substances such as sulfates, phosphates, sodium and the like, trace elements such as calcium, zinc and the like, and growth factors such as purine, pyrimidine and the like, as nutrients to the culture medium.
However, there has been a problem that when these nutrients are consumed upon growth of the microorganisms, the microorganisms produce in the medium short chain fatty acids, such as formic acid, acetic acid and the like, which are toxic to the growth of the microorganisms, and as a result, the growth of the microorganisms ceases.

In particular, *Escherichia coli,* which is frequently used for the production of recombinant proteins, is widely used as a host bacterium for high cell-density under aerobic conditions. In the high cell-density aerobic culture of *E. coli,* it is conventional to add an appropriate amount of glucose to the growth medium, but this causes to accumulation of short chain fatty acids mainly comprising acetic acid in the culture. These short chain fatty acids are main factors that inhibit high cell-density culture, and they also inhibit the production of recombinant proteins (see, for example, Non-Patent Document 1 and Non-Patent Document 2).

To address this problem, a fed-batch culture method in which nutrients are fed continuously or intermittently as the culture proceeds to the medium during culture (see, for example, Non-Patent Document 3), a dialysis culture method in which extracellular products are removed to the outside of the culture system using a dialysis membrane or the like (see, for example, Non-Patent Document 4), and others are employed for the culture of microorganisms.
By the fed-batch culture method, it is possible to arbitrarily control the concentration of a specific component in the medium, and for example, it is possible to maintain a sugar concentration in the medium within the optimal range for the cultured microorganism. Consequently, a desired microorganism can be efficiently cultured, and this method is widely employed. However, even by use of the fed-batch culture method, the production of organic acids cannot be suppressed, and there still remains several problems such as repression of growth rate, reduction in yield of recombinant proteins, and the like which are caused by the produced organic acids.
Furthermore, although the dialysis culture method allows the reduction of the influence of the produced short chain fatty acids by eliminating extracellular products, it also has problems such that specialized equipments are needed and the process is complicated.

In addition, in the glucose metabolism of *E*. *coli* under aerobic condition, when carbon sources are added in an amount exceeding the metabolizing capability of the TCA cycle, acetic acid is produced and released to the outside of the cells, and thereby cell growth and production of recombinant proteins are inhibited (see, for example, Non-Patent Document 5).
Since acetic acid is biosynthesized from acetyl-CoA by phosphotransacetylase (PTA) and acetic acid kinase (ACK), Non-Patent Document 5 describes the preparation of *E. coli* mutants that are deficient in the genes of these enzymes (PTA and ACK). However, in these mutants, although the biosynthetic pathway for acetic acid (PTA and ACK) is inactivated, still there are other pathways for the production of acetic acid. Thus, the amount of acetic acid produced was lowered, but the production was not completely repressed. Furthermore, the defective mutants excessively accumulated organic acids other than acetic acid, such as lactic acid, pyruvic acid and the like (see, for example, Non-Patent Document 6 and Non-Patent Document 7).
As such, development of microorganisms which are incapable of producing short chain fatty acids, such as formic acid, acetic acid and the like, which are produced during culture and are harmful to cell growth, has not yet been succeeded.
Meanwhile, in industrial production of these short chain fatty acids by microbial fermentation, development of an efficient method for producing the short chain fatty acids on a large scale is required. In this case, microorganisms showing sufficient tolerance to a short chain fatty acid would be needed, but such microorganisms have not been developed.

Meanwhile, a number of acetic acid bacterium-derived genes having a function of improving tolerance to acetic acid have been found by the present inventors, and among these genes included is a gene cluster comprising a motif of an ATP-binding cassette (see, for example, Patent Document 1).
These genes having the motif of an ATP-binding cassette are generically referred to as the ABC transporter family, and are assumed to encode proteins which serve as transporters having a function of transporting metabolites, drugs and the like from the inside of the cells to the outside of the cells, or from the outside of cells to the inside of cells.
When one of these genes constituting the ABC transporter family (ABC transporter gene) was ligated to a multicopy number vector and introduced into acetic acid bacterium, the resulting transformant (acetic acid bacterium) showed improved tolerance to acetic acid, but there was no influence on the tolerance to other organic acids (see, for example, Japanese Unexamined Patent Application Publication No. 2003-289868).

Patent Document 1: JP-A 2003-289868
Non-Patent Document 1: Appl. Environ. Microbiol., Vol. 56, p.1004-1011 (1990)
Non-Patent Document 2: Biotechnol. Bioeng., Vol. 39, p.663-671 (1992)
Non-Patent Document 3: Trends Biotechnol., Vol. 14, p.98-100 (1996)
Non-Patent Document 4: Appl. Microbiol. Biotechnol., Vol. 48, p.597-601 (1997)
Non-Patent Document 5: Biotechnol. Bioeng., Vol. 35, p.732-738 (1990)
Non-Patent Document 6: Biotechnol. Bioeng., Vol. 38, p.1318-1324 (1991)
Non-Patent Document 7: Biosci. Biotechnol. Biochem., Vol. 58, p.2232-2235 (1994)

### [Disclosure of the invention]

### [Problem to be solved by the invention]

Therefore, firstly, it is an object of the present invention to provide a method of conferring to microbial cells or the like tolerance to short chain fatty acids including formic acid and acetic acid which are harmful to the growth thereof, without suppressing the growth of the cells and with maintaining a high productivity of a useful substances.
Secondly, it is another object of the invention to provide a means for efficient culture of a microorganism in the presence of a short chain fatty acid, and a means for producing a useful short chain fatty acid via fermentation, with regard to the use of above-mentioned microorganism.

### [Means for solving the problem]

While conducting the study on a means to solve the above-described problems, the inventors of the present invention focused their attention on acetic acid, which is one of the short chain fatty acids, and took notice of an acetic acid bacterium-derived gene cluster which is assumed to belong to an ABC transporter family possessed by acetic acid bacteria, which confer high tolerance to acetic acid and is used for acetic acid fermentation in the manufacture of vinegar. And, the inventors extensively investigated as to whether the ABC transporter gene constituting the ABC transporter family can be introduced into and expressed in heterogeneous cells other than the original cells of acetic acid bacteria.
As a result, they discovered that transformants were also obtained from *Escherichia coli,* which belongs to a genus completely different from acetic acid bacteria and inherently possesses low ability to oxidize alcohol, or *Gluconacetobacter diazotrophicus,* which belongs to acetic acid bacteria but has low ability of acetic acid fermentation. They also unexpectedly discovered that, unlike acetic acid bacteria, the growth of transformants *of E. coli* or G *diazotrophicus* was not repressed by a short chain fatty acid (having 5 or fewer carbon atoms) other than acetic acid, that is, these bacteria showed tolerance to a short chain fatty acid. Furthermore, it was confirmed that introduction of the gene did not cause reduction in productivity of the useful substances whose productivity was the same as that of the original strain, or in productivity of recombinant proteins conferred by exogenously introduced gene.

Further, the inventors studied on the mechanism how the ABC transporter gene enhances tolerance to short chain fatty acid using transformants of acetic acid bacteria. Based on the finding that the intracellular acetic acid concentration of the transformant was reduced compared to that of the original strain, they confirmed that the transformant acquired an ability of transporting acetic acid from the inside of the cells to the outside of the cells, that is, to the medium. Therefore, it was assumed that this function could be applied as a mechanism for improving the tolerance and applied not only to acetic acid bacteria, but also to heterogeneous cells.

And, when a microorganism transformed with the gene was cultured at high cell-density, it can sufficiently grow even in the presence of a short chain fatty acid compared with a microorganism that was not transformed. This also leads to the finding that the transformant is useful for industrial culture.
Furthermore, it was also discovered that the concentration of an accumulated short chain fatty acid such as propionic acid and the like was increased.
The present invention is based on the above-stated findings.

Thus, the invention comprises the following (1) to (11).
(1) A method of breeding cells to improve tolerance to a short chain fatty acid, wherein a gene encoding a protein having a function of transporting a short chain fatty acid from the inside of cells to the outside of cells, is introduced and expressed in the cells.

(2) The method of breeding cells according to (1), wherein the gene encoding the protein having a function of transporting a short chain fatty acid from the inside of cells to the outside of cells is a DNA represented by the following (a) or (b):
(a) a DNA that comprises a nucleotide sequence consisting of nucleotide numbers 301 to 2073 in the nucleotide sequence set forth in SEQ ID NO: 1 of the Sequence Listing; or
(b) a DNA that comprises a nucleotide sequence which hybridizes with a probe comprising a nucleotide sequence consisting of nucleotide numbers 301 to 2073 in the nucleotide sequence set forth in SEQ ID NO: 1 of the Sequence Listing or comprising at least a part of said nucleotide sequence under stringent conditions, and that encodes a protein which is capable of enhancing tolerance to acetic acid.

(3) The method of breeding cells according to (1), wherein the gene encoding the protein having a function of transporting a short chain fatty acid from the inside of cells to the outside of cells is a DNA represented by the following (a) or (b):
(a) a DNA that comprises a nucleotide sequence consisting of nucleotide numbers 331 to 2154 in the nucleotide sequence set forth in SEQ ID NO: 3 of the Sequence Listing; or
(b) a DNA that comprises a nucleotide sequence which hybridizes with a probe comprising a nucleotide sequence consisting of nucleotide numbers 331 to 2154 in the nucleotide sequence set forth in SEQ ID NO: 3 of the Sequence Listing or comprising at least a part of said nucleotide sequence under stringent conditions, and that encodes a protein which is capable of enhancing tolerance to acetic acid.

(4) The method of breeding cells according to (1), wherein the gene encoding the protein having a function of transporting a short chain fatty acid from the inside of cells to the outside of cells is a DNA represented by the following (a), (b), (c) or (d):
(a) a DNA that comprises a nucleotide sequence consisting of nucleotide numbers 1724 to 2500 and a nucleotide sequence consisting of nucleotide numbers 1724 to 2500 in the nucleotide sequence set forth in SEQ ID NO:5 of in the Sequence Listing;
(b) a DNA that comprises both a nucleotide sequence which hybridizes with a probe comprising a nucleotide sequence consisting of nucleotide numbers 1002 to 1724 in the nucleotide sequence set forth in SEQ ID NO:5 of the Sequence Listing or comprising at least a part thereof under stringent conditions, and a nucleotide sequence consisting of nucleotide numbers 1724 to 2500 in the nucleotide sequence set forth in SEQ ID NO:5 of the Sequence Listing, and that encodes a protein complex which is capable of enhancing tolerance to acetic acid;
(c) a DNA that comprises both a nucleotide sequence consisting of nucleotide numbers 1002 to 1724 in the nucleotide sequence set forth in SEQ ID NO:5 of the Sequence Listing, and a nucleotide sequence which hybridizes with a probe comprising a nucleotide sequence consisting of nucleotide numbers 1724 to 2500 in the nucleotide sequence set forth in SEQ ID NO:5 of the Sequence Listing or comprising at least a part thereof under stringent conditions, and that encodes a protein complex which is capable of enhancing tolerance to acetic acid; or
(d) a DNA that comprises both a nucleotide sequence which hybridizes with a probe comprising a nucleotide sequence consisting of nucleotide numbers 1002 to 1724 in the nucleotide sequence set forth in SEQ ID NO:5 of the Sequence Listing or comprising at least a part thereof under stringent conditions, and a nucleotide sequence which hybridizes with a probe comprising a nucleotide sequence consisting of nucleotide numbers 1724 to 2500 in the nucleotide sequence set forth in SEQ ID NO:5 of the Sequence Listing or comprising at least a part thereof under stringent conditions, and that encodes a protein complex which is capable of enhancing tolerance to acetic acid.

(5) The method of breeding cells according to (1), wherein the gene encoding the protein having a function of transporting a short chain fatty acid from the inside of cells to the outside of cells is a DNA represented by the following (a) or (b):
(a) a DNA that comprises a nucleotide sequence consisting of nucleotide numbers 249 to 1025 in the nucleotide sequence set forth in SEQ ID NO: 8 of the Sequence Listing; or
(b) a DNA that comprises a nucleotide sequence which hybridizes with a probe comprising a nucleotide sequence consisting of nucleotide numbers 249 to 1025 in the nucleotide sequence set forth in SEQ ID NO: 8 of the Sequence Listing or comprising at least a part of said nucleotide sequence under stringent conditions, and that encodes a protein which is capable of enhancing tolerance to acetic acid.

(6) The method of breeding cells according to any one of (1) to (5), wherein the short chain fatty acid is formic acid, acetic acid, propionic acid, butyric acid, isobutyric acid, or valeric acid.
(7) Cells bred by the method according to any one of (1) to (5), wherein the cells are microbial cells.

(8) The cells according to (7), wherein the microbial cells are cells of acetic acid bacteria, bacteria belonging to the genus *Escherichia,* or genus *Bacillus.*
(9) A method for high cell-density culture that uses the cells according to (8).
(10) The method for high cell-density culture according to (9), wherein the cells are cultured in the presence of a short chain fatty acid.
(11) A method of preparing a fermentation broth comprising a short chain fatty acid, wherein the cells according to (8) are cultured under the conditions such that the cells produce a short chain fatty acid.

### [Effect of the invention]

According to the present invention, cells that are conferred tolerance to a short chain fatty acid and show improved tolerance to a short chain fatty acid can be bred. Furthermore, when the method of breeding of the invention is applied to microbial cells, cells whose growth is not affected by short chain fatty acids that are produced during culture and harmful to cell growth, can be efficiently obtained.
The microbial cells exhibiting tolerance to a short chain fatty acid obtained by the invention can also be applied to high cell-density culture in which short chain fatty acids are produced. Also, the cells can be used for the preparation of fermentation broth comprising short chain fatty acids at high concentrations. Particularly in the case of *Escherichia coli* to whose tolerance to a short chain fatty acid is conferred, or the like, the bacterial cells show significantly improved ability to grow in medium and can efficiently accumulate a high concentration of short chain fatty acids, thus being industrially useful.

### [Brief description of the drawings]

Fig. 1 is a schematic diagram showing a construction of *E. coli-Acetobacter* shuttle vector pGI18.
Fig. 2 is a set of graphs showing time courses in growth of the transformant and non-transformed strain according to Example 1 in (a) a medium with no addition, (b) a medium added with formic acid, (c) a medium added with acetic acid, and (d) a medium added with propionic acid.
Fig. 3 is a set of graphs showing time courses in growth of the transformant and non-transformed strain according to Example 1 in (a) a medium added with butyric acid, (b) a medium added with isobutyric acid, and (c) a medium added with n-valeric acid.
Fig. 4 is a set of graphs showing time courses in growth of the transformant and non-transformed strain according to Example 2 in (a) a medium added with formic acid, and (b) a medium added with acetic acid.
Fig. 5 is a schematic diagram showing a restriction enzyme map of a *Gluconacetobacter entanii-derived* gene fragment, the location of the gene conferring tolerance to short chain fatty acids, and the DNA fragment inserted into plasmid pABC31.
Fig. 6 is a set of graphs showing time courses in growth of the transformant and non-transformed strain according to Example 3 in (a) a medium added with formic acid, and (b) a medium added with acetic acid.
Fig. 7 is a set of graphs showing time courses in growth of the transformant and non-transformed strain according to Example 4 in (a) a medium added with formic acid, and (b) a medium added with acetic acid.
Fig. 8 is a schematic diagram showing a restriction enzyme map of a cloned *Gluconacetobacter entanii-derived* gene fragment, the location of the gene conferring tolerance to short chain fatty acids, and the DNA fragment inserted into plasmid pABC41.
Fig. 9 is a set of graphs showing time courses in growth of the respective cells according to Example 6(1), and time courses in amounts of total organic acids and acetic acid in the broth.
Fig. 10 is a graph showing time courses in growth in glucose fed-batch culture according to Example 6(2).
Fig. 11 is a graph showing time courses in growth of the transformant and non-transformed strain according to Example 7 in a medium comprising acetic acid.
Fig. 12 is a schematic diagram showing a restriction enzyme map of a DNA comprising a nucleotide sequence set forth in SEQ ID NO: 1 of the Sequence Listing, the location of the gene conferring tolerance to a short chain fatty acid, and the DNA fragment inserted into plasmid pABC1.

### [Best embodiment for carrying out the invention]

Hereinafter, the present invention will be described in detail.
An object of the invention is to provide a method of breeding cells which are transformed with a gene having a function of transporting a short chain fatty acid from the inside of cells to the outside of cells (gene conferring tolerance to short chain fatty acids), cells improved by the method of breeding, and a method for high cell-density culture using said cells, and a method of preparing a fermentation broth comprising a useful short chain fatty acid.

### [1] Method of breeding of the invention

The method of breeding cells to improve tolerance to a short chain fatty acid of the invention is characterized in that, as described in claim 1, a gene encoding a protein having a function of transporting a short chain fatty acid from the inside of cells to the outside of cells (gene conferring tolerance to short chain fatty acids) is introduced into and expressed in the cells.
Here, "protein having a function of transporting a short chain fatty acid from the inside of cells to the outside of cells" means a protein which exhibits, in cells of the present invention, a function of transporting a short chain fatty acid which is incorporated into the cells or produced by a intracellular activity such as metabolism or the like, to the outside of cells. For example, the protein means a protein that can reduce the intracellular acetic acid concentration by 15 to 20% or more compared to that of the parental strain, in a medium where acetic acid is added at the concentration affecting cell growth. Specific examples of such protein include a protein having an amino acid sequence set forth in SEQ ID NO: 2, 4 or 9 of the Sequence Listing, and protein complexes having amino acid sequences set forth in SEQ ID NOs.: 6 and 7.
Furthermore, proteins comprising mutations such as substitution, deletion, insertion, addition, inversion and the like of one or multiple, preferably one or a few, amino acids in the respective amino acid sequences set forth in SEQ ID NOs: 2, 4 and 9 of the Sequence Listing, are also included in the proteins mentioned above, as long as they have the function of transporting a short chain fatty acid from the inside of cells to the outside of cells. Protein complexes comprising mutations such as substitution, deletion, insertion, addition, inversion and the like of one or multiple, preferably one or a few, amino acids in any of the amino acid sequences set forth in SEQ ID NO: 6 and/or 7, are likewise included in the proteins mentioned above, as long as they have the function of transporting a short chain fatty acid from the inside of cells to the outside of cells.

Furthermore, the gene encoding the aforementioned protein (gene conferring tolerance to short chain fatty acids) means a gene which contains a coding region for the protein, and can be transformed into and expressed in cells. Representative examples of such genes include an acetic acid bacterium-derived ABC transporter genes forming a cluster which is assumed to belong to an ABC transporter family. An ABC transporter gene means a gene comprising a motif of an ATP-binding cassette, or a gene forming an operon with a gene comprising the motif which encodes a protein to form a protein complex. The gene comprising a motif of an ATP-binding cassette is generically referred to as the ABC transporter family, and is assumed to encode proteins serving as transporters, which have a function of transporting metabolites, drugs and the like from the inside of cells to the outside of cells, or from the outside of cells to the inside of cells.

Examples of such ABC transporter genes include a gene comprising a coding region encoding a protein having an amino acid sequence set forth in SEQ ID NO: 2, 4, 6, 7 or 9 of the Sequence Listing and specifically include DNAs comprising a nucleotide sequence consisting of nucleotide numbers 301 to 2073 of SEQ ID NO: 1, a nucleotide sequence consisting of nucleotide numbers 331 to 2154 of SEQ ID NO: 3, a nucleotide sequence consisting of nucleotide numbers 1002 to 1724 and a nucleotide sequence consisting of nucleotide numbers 1724 to 2500 of SEQ ID NO: 5, or a nucleotide sequence consisting of nucleotide numbers 249 to 1025 of SEQ ID NO: 8, in the nucleotide sequence set forth in SEQ ID NO: 1, 3, 5 or 8, as described in (a) of claims 2 to 5.
These DNAs may be ones that contain the specific nucleotide sequences, and it is also possible to use, for example, DNAs consisting of the full length of each of the nucleotide sequences set forth in SEQ ID NOs: 1, 3, 5 and 8 of the Sequence Listing.

These DNAs can be easily obtained by PCR using DNAs which are designed based on a nucleotide sequence set forth in SEQ ID NO: 1, 3, 5 or 8 as the primers and DNA of acetic acid bacteria (an acetic acid bacteria of genus *Acetobacter* or genus *Gluconacetobacter)* as the template, respectively. In particular, the DNAs consisting of the respective nucleotide sequences set forth in SEQ ID NOs: 1 and 3 of the Sequence Listing can be obtained from *Acetobacter aceti* strain No. 1023 (deposited at International Patent Organism Depositary, National Institute of Advanced Industrial Science and Technology (Central 6, 1-1, Higashi 1-chome, Tsukuba-shi, Ibaraki-ken, Japan) (former designation: Fermentation Research Institute Agency of Industrial Science and Technology, the Ministry of International Trade and Industry, former address: 1-3, Higashi 1-chome, Tsukuba-shi, Ibaraki-ken, Japan) under accession number FERM BP-2287 on June 27, 1983 (transferred from the original deposit on February 13, 1989)) as the template. Furthermore, the DNAs consisting of the respective nucleotide sequences set forth in SEQ ID NOs: 5 and 8 of the Sequence Listing can be respectively obtained from one species of *Gluconacetobacter entanii, Acetobacter altoacetigenes* strain MH-24 (deposited at International Patent Organism Depositary, National Institute of Advanced Industrial Science and Technology (Central 6, 1-1, Higashi 1-chome, Tsukuba-shi, Ibaraki-ken, Japan) (former title: Fermentation Research Institute Agency of Industrial Science and Technology, Ministry of International Trade and Industry, former address: 1-3, Higashi 1-chome, Tsukuba-shi, Ibaraki-ken, Japan) under accession number FERM BP-491 on February 23, 1984) as the template.

In addition, according to the invention, as described in (b) of claims 2, 3 and 5, of the nucleotide sequences set forth in SEQ ID NOs: 1, 3 and 8, a DNA that comprises a nucleotide sequence which hybridizes with a probe comprising a nucleotide sequence consisting of nucleotide numbers 301 to 2073 of SEQ ID NO: 1, a nucleotide sequence consisting of nucleotide numbers 331 to 2154 of SEQ ID NO: 3, or a nucleotide sequence containing at least a part of the foregoing nucleotide sequences under stringent conditions, and that encodes a protein which is capable of enhancing tolerance to acetic acid can be also used as the gene conferring tolerance to short chain fatty acid.
Furthermore, as described in (b), (c) or (d) of claim 4, (b), a DNA that comprises both a nucleotide sequence which hybridizes with a probe comprising a nucleotide sequence consisting of nucleotide numbers 1002 to 1724 in the nucleotide sequence set forth in SEQ ID NO: 5 of the Sequence Listing or comprising at least a part of the foregoing nucleotide sequence under stringent conditions, and a nucleotide sequence consisting of nucleotide numbers 1724 to 2500 in the nucleotide sequence set forth in SEQ ID NO: 5 of the Sequence Listing, and that encodes a protein complex which is capable of enhancing tolerance to acetic acid; (c) a DNA that comprises both a nucleotide sequence consisting of nucleotide numbers 1002 to 1724 in the nucleotide sequence set forth in SEQ ID NO: 5 of the Sequence Listing, and a nucleotide sequence which hybridizes with a probe comprising a nucleotide sequence consisting of nucleotide numbers 1724 to 2500 in the nucleotide sequence set forth in SEQ ID NO: 5 of the Sequence Listing or comprising at least a part of the foregoing nucleotide sequences under stringent conditions, and that encodes a protein complex which is capable of enhancing tolerance to acetic acid, or (d) a DNA that comprises both a nucleotide sequence which hybridizes with a probe comprising a nucleotide sequence consisting of nucleotide numbers 1002 to 1724 in the nucleotide sequence set forth in SEQ ID NO: 5 of the Sequence Listing or comprising at last a part of the foregoing nucleotide sequence under stringent conditions, and a nucleotide sequence which hybridizes with a probe comprising a nucleotide sequence consisting of nucleotide numbers 1724 to 2500 in the nucleotide sequence set forth in SEQ ID NO: 5 of the Sequence Listing or comprising at least a part of the foregoing nucleotide sequence under stringent conditions, and that encodes a protein complex which is capable of enhancing tolerance to acetic acid, can also be used likewise.

The term "stringent conditions" used herein refers to conditions where a so-called specific hybridization occurs, and a non-specific hybridization does not occur. Although it is difficult to precisely quantify these conditions, some examples can be presented, such as conditions under which DNAs having high homology with each other, for example, two DNAs having homology of 70% or greater with each other, can be hybridized, and two DNAs having homology lower than 70% with each other are not hybridized; conditions of conventional washing conditions for hybridization, for example, conditions under which washing is performed at 60°C with a solution, having a salt concentration corresponding to that of 1xSSC comprising 0.1% SDS and the like.

It can be confirmed that the above-mentioned gene conferring tolerance to short chain fatty acid encodes a protein having a function of transporting a short chain fatty acid from the inside of cells to the outside of cells by, for example, as described below in Examples, determining whether cells which are transformed with the gene and induced to express the gene, namely, transformants, can grow when cultured in a medium comprising a short chain fatty acid, or determining an increase in growth rate in that medium
Here, "short chain fatty acid" means a straight-chained or branched-chained fatty acid having 1 to 5 carbon atoms, where the fatty acid may or may not have unsaturated bonds. The cells obtained by the method of breeding of the invention are cells showing high tolerance to saturated a straight-chained or branched-chained fatty acids having 1 to 5 carbon atoms, namely, formic acid, acetic acid, propionic acid, butyric acid, isobutyric acid or valeric acid as described in claim 6.

Cells to which the method of breeding of the invention is applicable are not particularly limited, so long as cells are ones which can be transformed with a gene conferring tolerance to a short chain fatty acid, and express it. Examples of cells include microbial cells, including bacterial cells of *Escherichia coli, Bacillus subtilis,* Lactobacillus and the like; and yeast cells, the microbial cells belonging to genus *Aspergillus* and the like.
Among them, it is preferable to use microbial cells as the cells as described in claim 7. This is because culture efficiency in industrial production, particularly in high cell-density culture, and production efficiency in the production of a short chain fatty acid by fermentation are improved by enhancing tolerance to a short chain fatty acid.
Examples of the microbial cells include, in particular, as described in claim 8, the bacterial cells belonging to acetic acid bacteria, genus *Escherichia* or genus *Bacillus.* Among the bacterial cells, it is preferable to use those bacterial cells having essentially low ability to oxidize alcohol, such as *Escherichia coli,* acetic acid bacteria of genus *Gluconacetobacter,* and the like, because it is possible to significantly increase the amount of product or production efficiency of the desired short chain fatty acid and the cell growth.

Examples of acetic acid bacteria include those strains belonging to genera *Acetobacter* and *Gluconacetobacter.*
Specific examples of the bacteria belonging to genus *Acetobacter* include *Acetobacter aceti,* and in particular, *Acetobacter aceti* strain No. 1023 (deposited at International Patent Organism Depositary, National Institute of Advanced Industrial Science and Technology (Central 6, 1-1, Higashi 1-chome, Tsukuba-shi, Ibaraki-ken, Japan) (former designation: Fermentation Research Institute Agency of Industrial Science and Technology, Ministry of International Trade and Industry, former address: 1-3, Higashi 1-chome, Tsukuba-shi, Ibaraki-ken, Japan) under accession number FERM BP-2287 on June 27, 1983 (transferred from the original deposit deposited on February 13, 1989), *Acetobacter aceti* subspecies *xylinum* strain IF03288, *Acetobacter aceti* strain IFO3283, and the like.
Examples of the bacteria belonging to genus *Gluconacetobacter* include *Gluconacetobacter europaeus, Gluconacetobacter diazotrophicus,* and *Gluconacetobacter entanii,* and in particular, *Gluconacetobacter europaeus* strain DSM6160, *Gluconacetobacter diazotrophicus* strain ATCC49037, *Gluconacetobacter entanii, Acetobacter altoacetigenes* strain MH-24 (deposited at International Patent Organism Depositary, National Institute of Advanced Industrial Science and Technology (Central 6, 1-1, Higashi 1-chome, Tsukuba-shi, Ibaraki-ken, Japan) (former designation: Fermentation Research Institute Agency of Industrial Science and Technology, Ministry of International Trade and Industry, former address: 1-3, Higashi 1-chome, Tsukuba-shi, Ibaraki-ken, Japan) under accession number FERM BP-491 on February 23, 1984), which is one species of *Gluconacetobacter entanii,* can be used.

Preferred examples of coliform bacteria include the bacteria belonging to genus *Escherichia.*
Examples of the bacteria belonging to genus *Escherichia* include *Escherichia coli,* and in particular, *Escherichia coli* strain K12, *E. coli* strain JM109, *E. coli* strain DH5α, E. *coli* strain C600, *E. coli* strain BL21, *E. coli* strain W3110 and the like can be used.
Furthermore, examples of the bacteria belonging to genus *Bacillus* include *Bacillus subtilis* and *Bacillus subtilis (natto),* and in particular, *Bacillus subtilis* strain Marburg 168 and the like can be used.
Examples of the yeast cell that can be used include *Saccharomyces cerevisiae, Shizosaccharomyces pombe* and the like.
In particular, when DNA claimed in claim 2 or claim 3 of the invention is used as a gene conferring tolerance to a short chain fatty acid, it is preferable to use, among these cells, *Escherichia coli* or *Gluconacetobacter diazotrophicus* as the cells. *Escherichia coli* has inherently very low ability to oxidize alcohol, and *Gluconacetobacter diazotrophicus* has low ability of acetic acid fermentation even though it is an acetic acid bacterium. Thus, by conferring tolerance to a short chain fatty acid to these microorganisms, their usefulness can be sufficiently increased.

Transformation and expression of a gene conferring tolerance to a short chain fatty acid in cells according to the method of breeding of the invention can be performed using a recombinant vector. That is, this can be performed by a method of amplifying the intracellular copy number of the gene conferring tolerance to a short chain fatty acid by transforming the cells using a recombinant vector constructed by ligating the aforementioned gene to an appropriate vector; or by a method of amplifying the intracellular copy number of the gene conferring tolerance to a short chain fatty acid by transforming the cells using a recombinant vector in which a structural gene conferring tolerance to a short chain fatty acid and a promoter sequence which efficiently functions in the cells are ligated to an appropriate vector.

The vector that can be used for the construction of a recombinant vector may be a phage vector, which can autonomically propagate in the host, or a plasmid vector, or the like.
Examples of the plasmid vector include *Escherichia-*derived plasmids (for example, pBR322, pBR325, pUC118, pET16b, and the like), *Bacillus-derived* plasmids (for example, pUB110, pTP5, and the like), yeast-derived plasmids (for example, Yep13, Ycp50, and the like), and the like, while examples of the phage vector include λ phage (λgt10, λZAP, and the like), and the like.
Animal virus vectors such as those derived from retrovirus, vaccinia virus or the like, insect virus vectors such as those derived from baculovirus or the like, bacteria artificial chromosome (BAC), yeast artificial chromosome (YAC) and the like can be used to transform cells.

Also, multicopy vectors, transposons or the like can be used to introduce a desired DNA into the host, and according to the invention, such multicopy vectors or transposons are also to be included in the vectors of the invention.
Examples of the multicopy vectors include pUF106 (see, for example, Cellulose, p.153-158 (1989)), pMV24 (see, for example, Appl. Environ. Microbiol., Vol. 55, p.171-176 (1989)), pGI18 (see, for example, Production Example 1 described below), pTA5001(A), pTA5001(B) (see, for example, JP-A No. 60-9488), and the like. Furthermore, pMVL1, a chromosome-integration type vector (see, for example, Agric. Biol. Chem., vol. 52, p.3125-3129 (1988)), may also be included.
Examples of the transposon include Mu, IS 1452 and the like.

For construction of a recombinant vector by ligating the gene conferring tolerance to a short chain fatty acid to a vector, a method of cleaving purified DNA with an appropriate restriction enzyme, and inserting the resulting fragment into a restriction enzyme site or a multicloning site of an appropriate vector DNA to be ligated to the vector, or the like may be employed.
Here, the recombinant vector thus obtained is required to be expressed so as to produce a protein that is encoded by the gene conferring tolerance to a short chain fatty acid and has a function of transporting a short chain fatty acid from the inside of the cell to the outside of the cell, when transformed into a cell. Therefore, in addition to the gene conferring tolerance to a short chain fatty acid and the promoter sequence, if desired, cis-elements such as an enhancer and the like, a splicing signal, a poly(A) addition signal, a selection marker, a ribosome-binding sequence (SD sequence) and the like may also be inserted by ligating to the recombinant vector.
Here, examples of the selection marker include dihydrofolate reductase gene, kanamycin resistance gene, tetracycline resistance gene, ampicillin resistance gene, neomycin resistance gene, and the like.

In addition, the promoter sequence of the gene conferring tolerance to a short chain fatty acid on the chromosomal DNA may be substituted with another promoter sequence which efficiently functions in acetic acid bacteria belonging to genus *Acetobacter* or genus *Gluconacetobacter,* or in *Escherichia coli.* In this case, a recombinant vector with a DNA sequence homologous to chromosomal DNA may be prepared, and the constructed vector may be used to induce homologous recombination with the chromosome in a cell.
Examples of such promoter sequences include promoter sequences for the ampicillin resistance gene *of E. coli* plasmid pBR322 (TakaraBio, Inc.), the kanamycin resistance gene of plasmid pHSG298 (TakaraBio, Inc.), the chloramphenicol resistance gene of plasmid pHSG396 (TakaraBio, Inc.), β-galactosidase gene and the like, that are derived from microorganisms other than acetic acid bacteria.
Construction of a vector for homologous recombination is well known to those having ordinary skill in the art. As such, when an endogenous gene that confers tolerance to a short chain fatty acid is placed so as to be expressed under control of a potent promoter in a microorganism, the gene conferring tolerance to a short chain fatty acid is amplified due to multiple copies, and expression thereof is enhanced.

Specific examples of such recombinant vectors include pABC111, pABC112, pABC31 and pABC41, which are respectively obtained by ligating DNAs comprising the nucleotide sequences set forth in SEQ ID NO: 1, 3, 5 and 8 of the Sequence Listing to pGI18, an *Acetobacter-Escherichia coli* shuttle vector (multicopy vector).

Transformation and expression of the gene conferring tolerance to a short chain fatty acid in cells in the method of breeding of the invention can be performed by the standard methods, using the recombinant vectors described above. For example, in the case of bacterial cells, the method using calcium ions (see, for example, Agric. Biol. Chem., Vol. 49, p.2091-2097 (1985)), electroporation (see, for example, Proc. Natl. Acad. Sci., U.S.A., Vol. 87, p.8130-8134 (1990); Biosci. Biotech. Biochem., Vol. 58, p.974 (1994)), and the like may be used. In the case of using yeast cells as a host, for example, electroporation, a spheroplast method, a lithium acetate method and the like may be used.
Furthermore, the transformant may be selected by the properties conferred by the marker gene contained in the gene to be transformed. For example, in the case of using an ampicillin resistance gene as the marker gene, transformant can be obtained by selecting a cell exhibiting resistance to ampicillin. Specifically, a selection agar plate added with an appropriate amount of ampicillin (for example, about 100 µg/ml) is used, and cells are spread on it and cultured. Colonies that grow on the selection agar plate can be transformants. Also, in the case of using a neomycin resistance gene, a cell exhibiting resistance to the G418 drug can be selected.

According to the method of breeding of the invention, cells that show improved tolerance to a short chain fatty acid can be obtained by transformation of cells with a gene conferring tolerance to a short chain fatty acid and expressing the gene therein, as described above. In the case of bacterial cells, improvement of tolerance to short chain fatty acids of the bred cells can be confirmed as follows: culturing the cells in a medium added with about 0.01 to 3% of a short chain fatty acid for 15 hours or longer, determining the degree of growth by measuring the absorbance or dried cell mass, and comparing the cells with non-transformed original cells, whereby confirming that the transformant shows significantly increased cell mass compared with the original cell that exhibits no growth or insufficient growth. As described later in Examples, the present inventors have obtained cells showing improved tolerance to a short chain fatty acid, such as strain No.1023/pABC111 obtained by transforming *Acetobacter aceti* strain No. 1023 with the above-mentioned recombinant vector pABC 111; strain JM109/pABC 111 obtained by transforming *Escherichia coli* strain JM109 with the recombinant vector pABC111; strain ATCC49037/pABC 111 obtained by transforming *Gluconacetobacter diazotrophicus* strain ATCC49037 with the recombinant vector pABC111; strain JM109/pABC112 obtained by transforming *Escherichia coli* strain JM109 with the recombinant vector pABC112; strain JM109/pABC31 obtained by transforming *Escherichia coli* strain JM109 with the recombinant vector pABC31; and strain JM109/pABC41 obtained by transforming *Escherichia coli* strain JM109 with the recombinant vector pABC41. Five strains of these transformants are deposited at International Patent Organism Depositary, National Institute of Advanced Industrial Science and Technology (Central 6, 1-1, Higashi 1-chome, Tsukuba-shi, Ibaraki-ken, Japan), and their accession numbers are FERM BP-10184 for the strain JM109/pABC111; FERM BP-10186 for the strain ATCC49037/pABC111; FERM BP-10185 for the strain JM109/pABC112; FERM BP-10182 for the strain JM109/pABC31; and FERM BP-10194 for the strain JM109/pABC41, respectively.

### [2] High cell-density culture method of the invention

The high cell-density culture method of the invention is characterized in that, as described in claim 9, the cells described in claim 8 are used; in other words, bacterial cells belonging to acetic acid bacteria, genus *Escherichia* or genus *Bacillus* showing improved tolerance to short chain fatty acid obtained by introducing the gene conferring tolerance to a short chain fatty acid into bacterial cells and expressing the gene therein, according to the method of breeding of the invention described above in [1].
Here, "high cell-density culture method" means a method of culturing bacterial cells to a high density, that is, to the cell density of 50 to 200 g (dry cell)/L in the medium. The medium, culture period and culture conditions may be appropriately selected according to the type of the cell, or the like. For example, in the case of *Escherichia coli,* any of complex and synthetic mediums may be used, and for example, the cells can be cultured under aerobic condition in a glucose-added LB medium at 28 to 37°C.
When nutrients in the medium are completely consumed by the bacterial cells, short chain fatty acids are usually produced in culture broth, that causes repression of growth. However, the high cell-density culture method of the invention employs bacterial cells showing improved tolerance to a short chain fatty acid, and thus can prevent repression of growth. Thus, as described in claim 10, even though microbial cells are cultured in the presence of a short chain fatty acid such as formic acid, acetic acid, propionic acid, butyric acid, isobutyric acid, valeric acid or the like which are toxic to cell growth, the growth is not repressed, and the productivity of useful substances produced by the microorganism or recombinant proteins encoded by introduced exogenous gene can be maintained. Here, the useful substance may be any substance that can be produced by microorganisms, and may be in any form and not particularly limited. One example thereof may be the cells of a microorganism itself. Furthermore, when the cells of the invention are used, the final cell density can be increased, and thus any substance, even it is a product as a result of normal metabolic activity, can be produced very efficiently. Examples of such substance include fatty acids, amino acids, antibiotics, enzymes, vitamins, alcohol, and the like. As shown in Example 6, the amount of organic acids produced in the medium can be increased. Also, examples of the recombinant protein include human growth hormones or peptides, interleukin 1β, interferon, and the like.
Particularly, in the case of *Escherichia coli,* organic acids other than the short chain fatty acids having 5 or fewer carbon atoms, such as citric acid, malic acid, succinic acid, pyroglutamic acid and the like, can be efficiently produced.

Examples of the high cell-density culture method, which is used in various kinds of application from laboratory scale to industrial production scale, include fed-batch culture and dialysis culture.
The fed-batch culture is a method of culture cells while continuously or intermittently feeding specific nutrients to the medium during the culture period. The nutrients to be fed are preferably carbon sources such as glucose, glycerol and the like, which are incorporated and directly used as raw materials for production of short chain fatty acids that inhibit cell growth, or protein production. In order to maintain a constant concentration of the carbon sources in the medium and/or growth rate, feeding may be carried out in a manner where a feeding rate is increased exponentially as the culture proceeds or in a manner where a feeding rate is increased step-wisely (generally at an interval of 2 to 5 hours) with maintaining the concentration of the carbon source in a medium below a predetermined concentration. The culture conditions such as medium composition, culture temperature, humidity, pH, culture time, stirring, and the like may be appropriately selected depending on strain or state of the bacterial cell.
In the conventional methods of fed-batch culture, it was necessary to control the feeding rate of glucose or the like and to control the concentration of dissolved oxygen in the medium minutely for suppression of production of short chain fatty acids, to avoid inhibition of cell growth, repression of protein production or the like, caused by the short chain fatty acids including acetic acid, that are produced during the culture of microorganisms. However, the bacterial cells belonging to acetic acid bacteria, genus *Escherichia* or genus *Bacillus,* in which cells show improved tolerance to a short chain fatty acid according to the invention, can maintain productivity without being affected by the short chain fatty acids, and thus the laborious control in the conventional method can be unnecessary.

In addition, the dialysis culture is a method of performing culture while removing extracellular products such as acetic acid to the outside of the culture system by use of dialysis membrane or the like.
While conventional methods of dialysis culture require specialized equipment, the bacterial cells belonging to acetic acid bacteria, genus *Escherichia* or genus *Bacillus,* in which cells show improved tolerance to a short chain fatty acid according to the invention, can maintain productivity without being affected by the short chain fatty acids, and thus the culture can be performed using a simple equipment, for example, ajar fermentor.

### [3] Preparation of fermentation broth comprising short chain fatty acids

The method of preparing a fermentation broth according to the invention is characterized in that the cells as described in claim 8 are cultured under the conditions where the cells produce a short chain fatty acid.
The cells as described in claim 8 refer to the bacterial cells belonging to acetic acid bacteria, genus *Escherichia* or genus *Bacillus,* in which cells show improved tolerance to a short chain fatty acid, and are obtained by introducing the gene conferring tolerance to a short chain fatty acid into bacterial cells which belong to acetic acid bacteria, genus *Escherichia* or genus *Bacillus,* expressing the gene therein and culturing the cells, according to the method of breeding of the invention described above in [1]. Among these cells, those bacterial cells having an ability to produce a desired short chain fatty acid can be selected and used. For example, *Escherichia* can produce all types of short chain fatty acids, while acetic acid bacteria can selectively produce acetic acid. Furthermore, the short chain fatty acids also include lactic acid, in addition to formic acid, acetic acid, propionic acid, butyric acid, isobutyric acid, and valeric acid.

According to the method of preparing a fermentation broth of the invention, the bacterial cells of the present invention need to be cultured under the conditions where the bacterial cells produce short chain fatty acids, and such conditions may be suitably selected depending on the type of the bacterial cell or the type of the short chain fatty acid to be produced. Generally, a short chain alcohol corresponding to the short chain fatty acid is added to the culture in an appropriate amount.

### [Examples]

Hereinafter, the present invention will be described in detail with reference to Examples.

### (Production Example 1) Development of Escherichia coli-Acetobacter shuttle vector pGI18

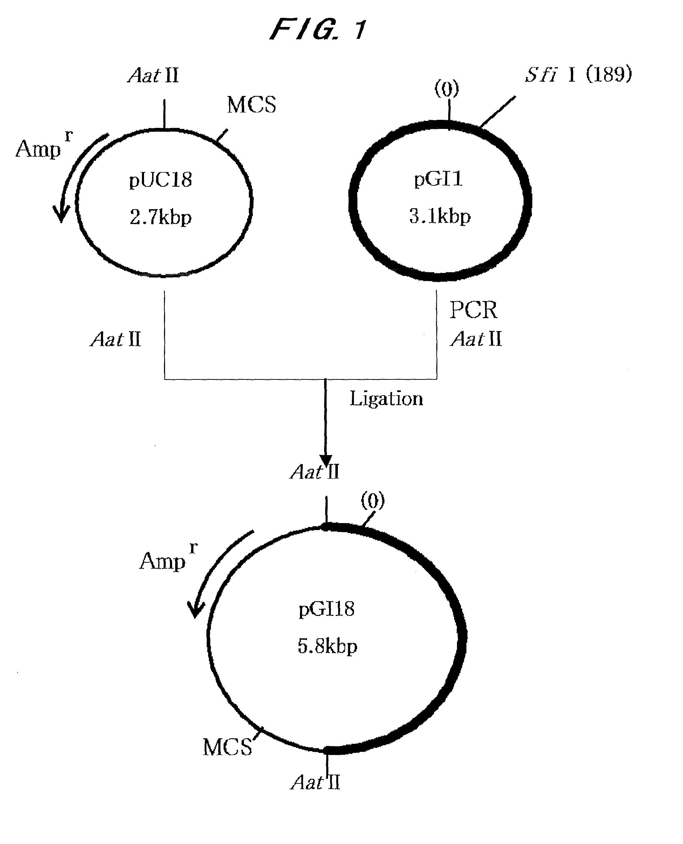

*Escherichia coli-Acetobacter* shuttle vector pGI18 was constructed from pGI1, a plasmid of about 3.1 kb derived *Acetobacter altoacetigenes* strain MH-24 (FERM BP-491), and pUC 18. Figure 1 presents a scheme for the construction of the plasmid pGI18.

First, plasmid pGI1 was prepared from *Acetobacter altoacetigenes.*
That is, the bacteria cells were collected from a culture broth of the *Acetobacter altoacetigenes* strain MH-24, and the cells were lysed using sodium hydroxide or sodium dodecyl sulfate. Subsequently, the resulting lysate was treated with phenol, and the plasmid DNA was purified using ethanol.
The obtained plasmid was a ring-shaped double-stranded DNA having three recognition sites for HincII and one recognition site for SfiI, and the total length of the plasmid was about 3100 nucleotide pairs (3.1 kbp). There were no recognition sites for EcoRI, SacI, KpnI, SmaI, BamHI, XbaI, SalI, PstI, SphI and HindIII in the plasmid. This plasmid was designated as pGI1 and was used for the construction of the vector pGI18.

The plasmid pGI1 thus obtained was amplified by PCR and the PCR product was cleaved by AatII. The fragment thus obtained was inserted into the cleavage site of pUC18 (2.7 kbp, Takara Bio, Inc.) digested with AatII to construct the plasmid pGI18.
In detail, the PCR was performed as follows. The plasmid pGI1 was used as the template, and primer A (see the nucleotide sequence described in SEQ ID NO: 10 of the Sequence Listing) and primer B (see the nucleotide sequence described in SEQ ID NO: 11 of the Sequence Listing), both comprising recognition site for restriction enzyme AatII, were used as primers. Thirty cycles of the PCR was performed using the template and primers described above and KOD-Plus (Toyobo Co., Ltd.), each cycle consisting of heating at 94°C for 30 seconds, 60°C for 30 seconds and 68°C for 3 minutes.
The resulting plasmid pGI18 contained, as shown in Fig. 1, both pUC18 and pGI1, and the full length was about 5800 nucleotide pairs (5.8 kbp).
The nucleotide sequence of this plasmid pGI18 was shown in SEQ ID NO: 12 of the Sequence Listing.

### (Example 1) Transport of acetic acid by Acetobacter aceti transformed with DNA comprising nucleotide sequence set forth in SEQ ID NO: 1 of Sequence Listing

### (1) Preparation of transformant

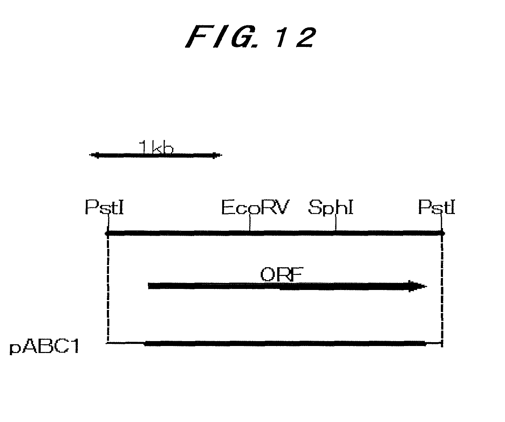

pABC 1 (Fig. 12) into which a DNA comprising the nucleotide sequence set forth in SEQ ID NO: 1 of the Sequence Listing was inserted, was cleaved with restriction enzyme PstI, and the fragment of about 2.5 kb thus obtained was ligated to the restriction enzyme PstI cleavage site of the *Acetobacter- E. coli* shuttle vector pGI18 prepared in the Production Example 1, to construct a plasmid pABC 111.
The pABC 111 thus constructed was used to transform *Acetobacter aceti* strain No. 1023 (FERM BP-2287) by electroporation (See Proc. Natl. Acad. Sci. U.S.A., Vol. 87, p.8130-8134 (1990)). The transformant was selected on YPG medium added with 100 µg/ml of ampicillin and 2% of acetic acid.
The plasmid DNA was extracted from cells of the ampicillin-resistant transformant which were cultured in the above mentioned selection medium and analyzed by the standard method, and it was confirmed that the transformed cells carried a plasmid comprising the DNA comprising the nucleotide sequence set forth in SEQ ID NO: 1 of the Sequence Listing.

### (2) Acetic acid transport in transformant

The resulting transformant (strain No. 1023/pABCl 11) carrying the plasmid pABC111 and showing tolerance to ampicillin was grown in YPG medium added with acetic acid, and was compared with *Acetobacter aceti* strain No. 1023 (strain No.1023/pGI18) transformed with the shuttle vector pGI18, in terms of the intracellular acetic acid concentration.
That is, strain No.1023/pABC111 and strain No.1023/pGI18 were cultured in 100 ml each of YPG medium added with potassium acetate at concentrations of 1, 2, 3 or 4% (w/v), according to the method of Stainer et al. (see, for example, Biotechnol. Bioeng., Vol. 84, p.40-44, 2003). The bacterial cells were collected from 50 ml of the culture broth and lysed with alkali, and then the intracellular acetic acid concentration (M) of cells obtained from each culture was measured using F-kit (Roche) and compared.
The results of the intracellular potassium acetate concentration in each culture are presented in Table 1.

**[Table 1]**

| Potassium acetate concentration (%(w/v)) | 1 | 2 | 3 | 4 |
|---|---|---|---|---|
| Strain No.1023/pGI18 | 1.27 M | 3.41 M | 5.31 M | 7.80 M |
| Strain No.1023/pABC111 | 1.24 M | 3.16 M | 4.25 M | 6.59 M |

As shown in Table 1, the intracellular acetate concentration of the transformant strain No. 1023/pABC111 and the non-transformed strain No.1023/pGI18 both increased with increasing potassium acetate concentration. However, the degree of increase was relatively low in the transformant (strain No.1023/pABC 111), and in particular, in the presence of 4% (w/v) of potassium acetate, strain No.1023/pABC111 accumulated acetate inside the cell, the amount of which was only 84% of that of the strain No.1023/pGI18. This shows that the intracellular acetic acid was transported to the outside of the bacterial cells in the transformant strain No. 1023/pABC 111.
From the results, it was confirmed that the DNA comprising the nucleotide sequence set forth in SEQ ID NO: 1 of the Sequence Listing was a gene encoding a protein with a function of transporting acetic acid from the inside of the cells to the outside of the cells.

### (Example 2) Tolerance to short chain fatty acid of E. coli transformed with DNA comprising nucleotide sequence set forth in SEQ ID NO: 1 of Sequence Listing

### (1) Preparation of transformant

*E. coli* strain JM109 was transformed with pABC111 constructed in Example 1 by electroporation according to the standard method, and the transformant was selected on the LB agar medium added with 100 µg/ml of ampicillin.
From cells of the ampicillin-resistant transformant, which had been grown in the selective medium, the plasmid DNA was extracted and analyzed by the standard method, and it was confirmed that the transformant carried a plasmid comprising the gene conferring tolerance to acetic acid.
The resulting transformant (strain JM109/pABC 111) was deposited at International Patent Organism Depositary, National Institute of Advanced Industrial Science and Technology (Central 6, 1-1, Higashi 1-chome, Tsukuba-shi, Ibaraki-ken, Japan) on December 14, 2004 under Deposit No. FERM BP-10184.

### (2) Tolerance to short chain fatty acid of transformant

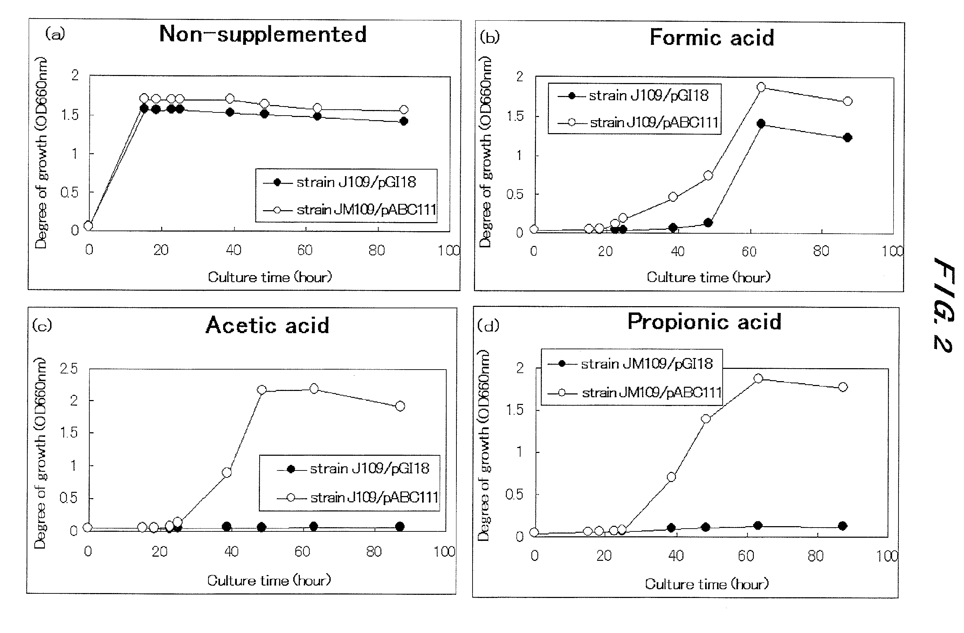

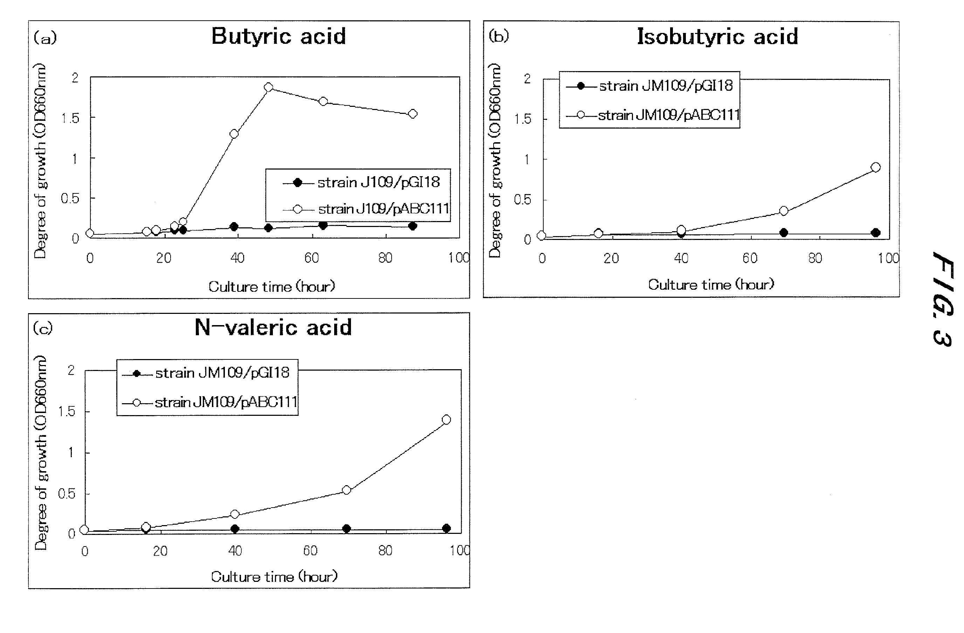

The resulting ampicillin-resistant transformant (strain JM109/pABC111), which carried the plasmid pABC111, was compared with *E. coli* (strain JM109/pGI18) carrying the shuttle vector pGI18 in terms of the growth in the LB medium which was added with formic acid, acetic acid, propionic acid, butyric acid, isobutyric acid, or valeric acid, respectively as a short chain fatty acid.
That is, strain JM109/pABC111 and strain JM109/pGI18 were cultured with shaking (150 rpm) in a LB medium (pH 5.0: a medium added with a short chain fatty acid) comprising any one of formic acid 0.10%, acetic acid 0.15%, propionic acid 0.10%, butyric acid 0.10%, isobutyric acid 0.15%, and n-valeric acid 0.25% as a short chain fatty acid, 100 µg/ml of ampicillin, and 1 mM IPTG (isopropyl-1-thio-β-D-galactopyranoside) at 37°C. Further, for comparison, they were cultured similarly as described above, except that a short chain fatty acid was not added to the medium. In each medium, absorbance of the broth, indicative of the growth (cell mass), was measured at 660 nm during culture and the measured values of absorbance were compared. Figs. 2 and 3 show the time course in growth (absorbance at 660 nm) during culture in each medium.

As shown in Figs. 2 and 3, both cells grew in the non-supplemented medium, on the other hand, strain JM109/pGI18 which did not carry the subject gene did not grow, or not substantially grow in the medium added with a short chain fatty acid, but the strain JM109/pABC111 which was transformed with the subject gene grew in the medium added with a short chain fatty acid. That is, the growth of non-transformed strain (strain JM109/pGI18) was affected by the presence of the short chain fatty acid, but the transformant (strain JM109/pABC111) was tolerant to all the short chain fatty acids tested.
This demonstrates that *E*. *coli* showing improved tolerance to various short chain fatty acids can be obtained by introducing DNA comprising the nucleotide sequences set forth in SEQ ID NO: 1 of Sequence Listing into the cells.

### (Example 3) Tolerance to short chain fatty acid of E. coli transformed with DNA comprising nucleotide sequence set forth in SEQ ID NO: 3 of Sequence Listing

### (1) Preparation of transformant

The DNA comprising the nucleotide sequences set forth in SEQ ID NO: 3 of Sequence Listing was amplified by a PCR process, and the obtained fragment was inserted into the site cleaved by restriction enzyme SmaI cleavage site of the *Acetobacter-E. coli* shuttle vector pGI18 prepared as in Production Example 1, to construct a plasmid pABC112.
Specifically, the PCR process was performed as follows. The genomic DNA of *Acetobacter altoacetigenes* strain MH-24 (FERM BP-491) was used as the template, and primer 1 (see the nucleotide sequence set forth in SEQ ID NO: 13 of Sequence Listing) and primer 2 (see the nucleotide sequence set forth in SEQ ID NO: 14 of Sequence Listing) were used as primers. Thirty cycles of PCR was performed using the template and primers described above and KOD-Plus (Toyobo Co., Ltd.), each cycle consisting of heating at 94°C for 15 seconds, 60°C for 30 seconds, and 68°C for 2 minutes.

*E. coli (Escherichia coli)* stain JM109 was transformed with the resulting pABC112 by electroporation (see Biosci. Biotech. Biochem., Vol. 58, p. 974 (1994)). The transformant was selected on the LB agar medium added with 100 µg/ml of ampicillin.
The plasmid DNA was extracted from cells of the ampicillin-resistant transformant which were cultured in the above mentioned selection medium and analyzed by the standard method, and it was confirmed that the transformant carried a plasmid comprising the DNA comprising the nucleotide sequence set forth in SEQ ID NO: 3 of Sequence Listing.
The resulting transformant (strain JM109/pABC112) was deposited at International Patent Organism Depositary, National Institute of Advanced Industrial Science and Technology (Central 6, 1-1, Higashi 1-chome, Tsukuba-shi, Ibaraki-ken, Japan) on December 14, 2004 under Deposit No. FERM BP-10185.

### (2) Tolerance to short chain fatty acid of transformant

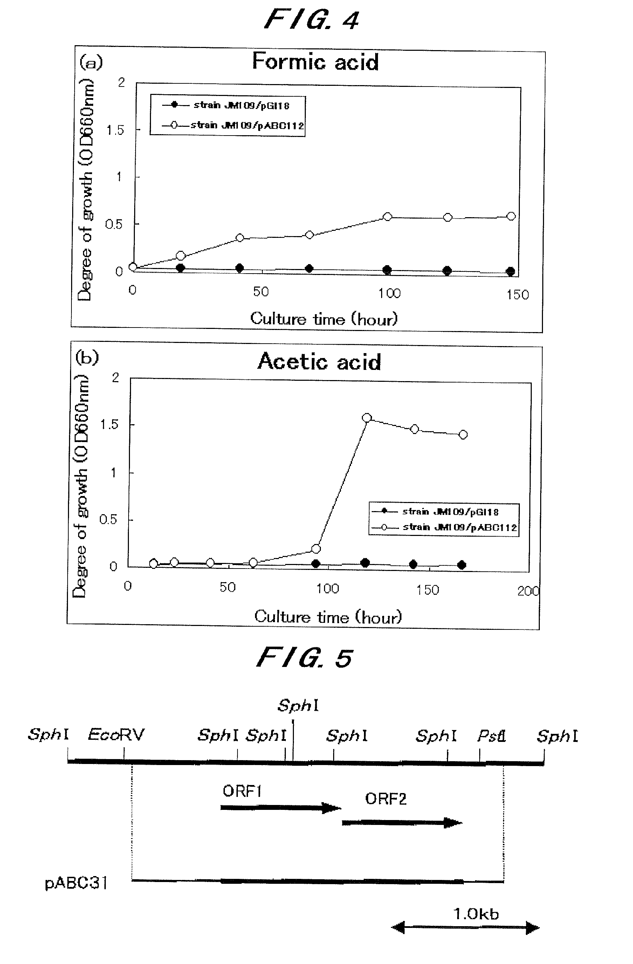

The resulting ampicillin-resistant transformant (strain JM109/pABC112), which carried the plasmid pABC112, was compared with *E. coli* (strain JM109/pGI18) carrying the shuttle vector pGI18 in terms of the growth in the LB medium added with formic acid or acetic acid as a short chain fatty acid.
Specifically, strain JM109/pABC112 and strain JM109/pGI18 were cultured without or with shaking (150 rpm) in a LB medium (pH 5.0) comprising 0.10% of formic acid, or 0.15% of acetic acid as a short chain fatty acid, 100 µg/ml of ampicillin, and 1 mM IPTG (isopropyl-1-thio-β-D-galactopyranoside) at 37°C. In each medium added with a short chain fatty acid, absorbance of the broth, indicative of the growth (cell mass), was measured at 660 nm during culture and the measured values of absorbance were compared. Fig. 4 shows the time course in growth (absorbance at 660 nm) during culture in each medium.

As shown in Fig. 4, strain JM109/pABC112 which was the transformant grew in any of the media added with a short chain fatty acid, but strain JM109/pABC18 which did not carried the subject gene did not grew in both media added with a short chain fatty acid. That is, the growth of non-transformed strain (strain JM109/pGI18) was affected by the presence of the short chain fatty acid, but the transformant (strain JM109/pABC112) showed tolerance to both formic acid and acetic acid.
This demonstrates that *E*. *coli* showing improved tolerance to a short chain fatty acids can be obtained by introducing DNA comprising the nucleotide sequences set forth in SEQ ID NO: 3 of Sequence Listing into the cells of *E*. *coli.*

### (Example 4) Tolerance to short chain fatty acid of E. coli transformed with DNA comprising nucleotide sequence set forth in SEQ ID NO: 5 of Sequence Listing

### (1) Preparation of E. coli transformant

The DNA comprising the nucleotide sequences set forth in SEQ ID NO: 5 of Sequence Listing was amplified by a PCR process, and the obtained fragment was ligated into the site cleaved by restriction enzyme SmaI cleavage site of the *Acetobacter-E. coli* shuttle vector pGI18 prepared in Production Example 1, to construct a plasmid pABC31.
Specifically, the PCR process was performed as follows. The genomic DNA of *Acetobacter altoacetigenes* strain MH-24 (FERM BP-491) was used as the template, and primer 3 (see the nucleotide sequence set forth in SEQ ID NO: 15 of Sequence Listing), and primer 4 (see the nucleotide sequence set forth in SEQ ID NO: 16 of Sequence Listing) were used as the primers. Thirty cycles of PCR was performed using the template and primers described above and KOD-Plus (Toyobo Co., Ltd.), each cycle consisting of heating at 94°C for 15 seconds, 60°C for 30 seconds, and 68°C for 1 minute.
For the genomic DNA of *Acetobacter altoacetigenes* strain MH-24, the restriction enzyme map of the nucleotide sequence set forth in SEQ ID NO: 5, the position of the nucleotide sequence set forth in SEQ ID NO: 5, and the schematic diagram of the fragment inserted into the plasmid pABC31 are presented in Fig. 5.

*E. coli (Escherichia coli)* strain J M109 was transformed with the resulting pABC31 by electroporation. The transformant was selected on the LB agar medium added with 100 µg/ml of ampicillin.
The plasmid DNA was extracted from cells of the ampicillin-resistant transformant which were cultured in the above mentioned selection medium and analyzed by the standard method. It was confirmed that the transformant carried a plasmid comprising DNA comprising the nucleotide sequence set forth in SEQ ID NO: 5 of Sequence Listing.
The resulting transformant (strain JM109/pABC31) was deposited at International Patent Organism Depositary, National Institute of Advanced Industrial Science and Technology (Central 6, 1-1, Higashi 1-chome, Tsukuba-shi, Ibaraki-ken, Japan) on December 14, 2004 under Deposit No. FERM BP-10182.

### (2) Tolerance to short chain fatty acid of transformant

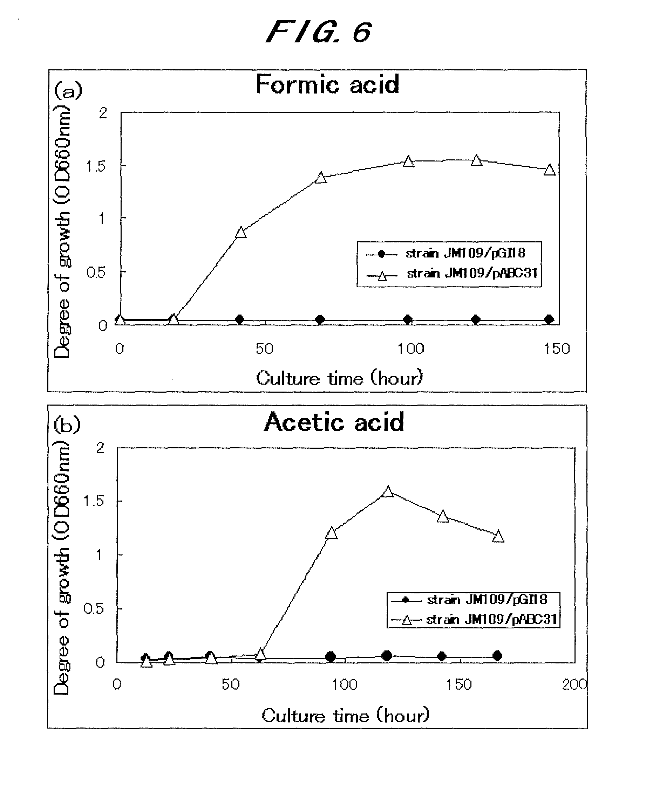

The resulting ampicillin-resistant transformant (strain JM109/pABC31), which carried the plasmid pABC31, was compared with *E*. *coli* (strain JM109/pGI18) carrying the shuttle vector pGI18 in terms of the growth in the LB medium added with formic acid or acetic acid as a short chain fatty acid.
Specifically, strain JM109/pABC31 and strain JM109/pGI18 were cultured without or with shaking (150 rpm) in a LB medium (pH 5.0) added with 0.10% of formic acid or 0.15% of acetic acid as a short chain fatty acid, 100 µg/ml of ampicillin, and 1 mM IPTG (isopropyl-1-thio-β-D-galactopyranoside) at 37°C. In each medium added with a short chain fatty acid, absorbance of the broth, indicative of the growth (cell mass), was measured at 660 nm during culture and the measured values of absorbance were compared. Fig. 6 shows the time course in growth (absorbance at 660 nm) during culture in each medium.

As shown in Fig. 6, strain JM109/pABC31 that was the transformant grew in any of the media added with a short chain fatty acid, but the *E. coli* strain JM109/pGI18 did not grow in the mediums added with short chain fatty acid. That is, the growth of non-transformed strain (strain JM109/pGI18) was affected by the presence of the short chain fatty acid, but the transformant (strain JM109/pABC31) showed tolerance to both formic acid and acetic acid.
This demonstrates that *E*. *coli* showing improved tolerance to a short chain fatty acid can be obtained by introducing DNA comprising the nucleotide sequences set forth in SEQ ID NO: 5 of Sequence Listing into the cells of *E. coli.*

### (Example 5) Tolerance to short chain fatty acid of E. coli transformed with DNA comprising nucleotide sequence set forth in SEQ ID NO: 8 of Sequence Listing

### (1) Preparation of E. coli transformant

The DNA comprising the nucleotide sequences set forth in SEQ ID NO: 8 of Sequence Listing was amplified by a PCR process, and the obtained fragment was ligated into the site cleaved by restriction enzyme SmaI cleavage site of the *Acetobacter-E. coli* shuttle vector pGI18 prepared in Production Example 1, to construct a plasmid pABC41.
Specifically, the PCR process was performed as follows. The genomic DNA of *Acetobacter altoacetigenes* strain MH-24 (FERM BP-491) was used as the template, and primer 5 (see the nucleotide sequence set forth in SEQ ID NO: 17 of Sequence Listing) and primer 6 (see the nucleotide sequence set forth in SEQ ID NO: 18 of Sequence Listing) were used as the primers. Thirty cycles of PCR were performed using the template and primers described above and KOD-Plus (Toyobo Co., Ltd.), each cycle consisting of heating at 94°C for 15 seconds, 60°C for 30 seconds, and 68°C for 1 minute.

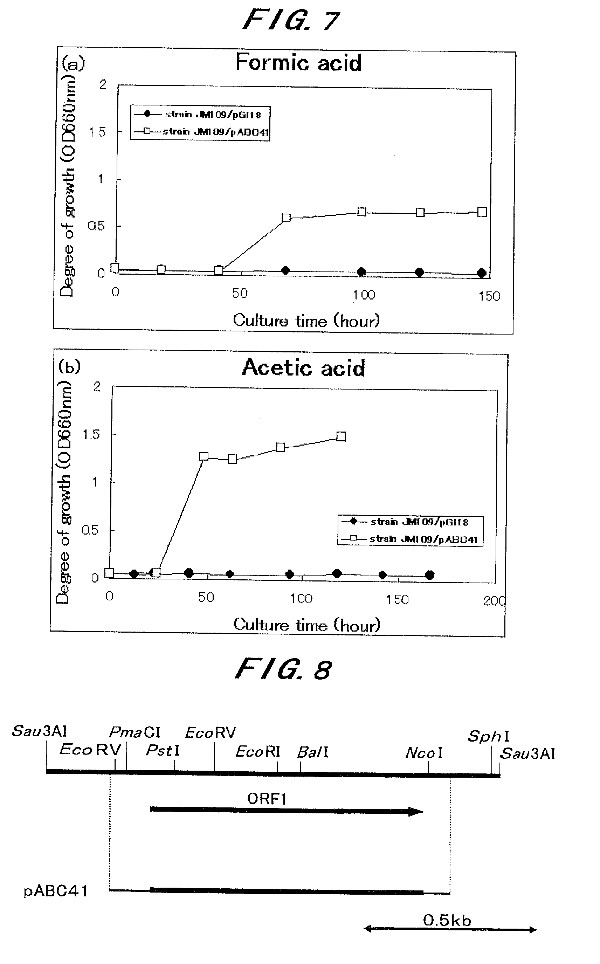

*E. coli (Escherichia coli)* strain JM109 was transformed with the resulting pABC41 by electroporation. The transformant was selected on the LB agar medium added with 100 µg/ml of ampicillin.
The plasmid DNA was extracted from cells of the ampicillin-resistant transformant which were cultured in the above mentioned selection medium and analyzed by the standard method, and it was confirmed that the transformant carried a plasmid comprising the DNA comprising the nucleotide sequence set forth in SEQ ID NO: 8 of Sequence Listing.
It was confirmed that there was an open-reading-frame (ORF) encoding the amino acid sequence consisting of 259 amino acids set forth in SEQ ID NO: 9, from the nucleotide numbers 249 through 1025, among the nucleotide sequences set forth in SEQ ID NO: 8 of Sequence Listing. Further, the homology of the amino acid sequence set forth in SEQ ID NO: 9 of Sequence Listing with known sequences was as low as 30% at most, indicating that the gene comprising the nucleotide sequences set forth in SEQ ID NO: 8 of Sequence Listing was a novel gene, first discovered by the present inventors.
For the genomic DNA of *Acetobacter altoacetigenes* MH-24 strain, the restriction enzyme map of the nucleotide sequence set forth in SEQ ID NO: 8, the position of the nucleotide sequence set forth in SEQ ID NO: 8, and the schematic diagram of the fragment inserted into the plasmid pABC41 are presented in Fig. 8.
The resulting transformant (strain JM109/pABC41) was deposited at International Patent Organism Depositary, National Institute of Advanced Industrial Science and Technology (Central 6, 1-1, Higashi 1-chome, Tsukuba-shi, Ibaraki-ken, Japan) on December 27, 2004 under Deposit No. FERM BP-10194.

### (2) Tolerance to short chain fatty acid of transformant

The resulting ampicillin-resistant transformant (strain JM109/pABC41), which carried the plasmid pABC41, was compared with *E. coli* (strain JM109/pGI18) carrying the shuttle vector pGI18, in terms of the growth in the LB medium added with formic acid or acetic acid as a short chain fatty acid.
Specifically, the strain JM109/pABC41 and the strain JM109/pGI18 were cultured without or with shaking (150rpm) in a LB medium (pH 5.0) comprising 0.15% of formic acid or acetic acid as a short chain fatty acid, 100 µg/ml of ampicillin, and 1 mM IPTG (isopropyl-1-thio-β-D-galactopyranoside) at 37°C. In each medium added with a short chain fatty acid, absorbance of the broth, indicative of the growth (cell mass), was measured at 660 nm during culture and the measured values of absorbance were compared. Fig. 7 shows the time course in growth (absorbance at 660 nm) during culture in each medium.

As shown in Fig. 7, the strain JM109/pABC41 which was a transformant grew in any of the media added with a short chain fatty acid, but the *E. coli* strain JM109/pGI18 did not grow in the medium added with a short chain fatty acid. That is, the growth of the non-transformed strain (strain JM109/pGI18) was affected by the presence of the short chain fatty acid, but the transformant (strain JM109/pABC41) showed tolerance to both formic acid and acetic acid.
This demonstrates that *E*. *coli* showing improved tolerance to a short chain fatty acid can be obtained by introducing DNA comprising the nucleotide sequences set forth in SEQ ID NO: 8 of Sequence Listing into the cells of *E*. *coli.*

### (Example 6) High cell-density culture of E. coli transformant

### (1) Culture in glucose-supplemented LB medium

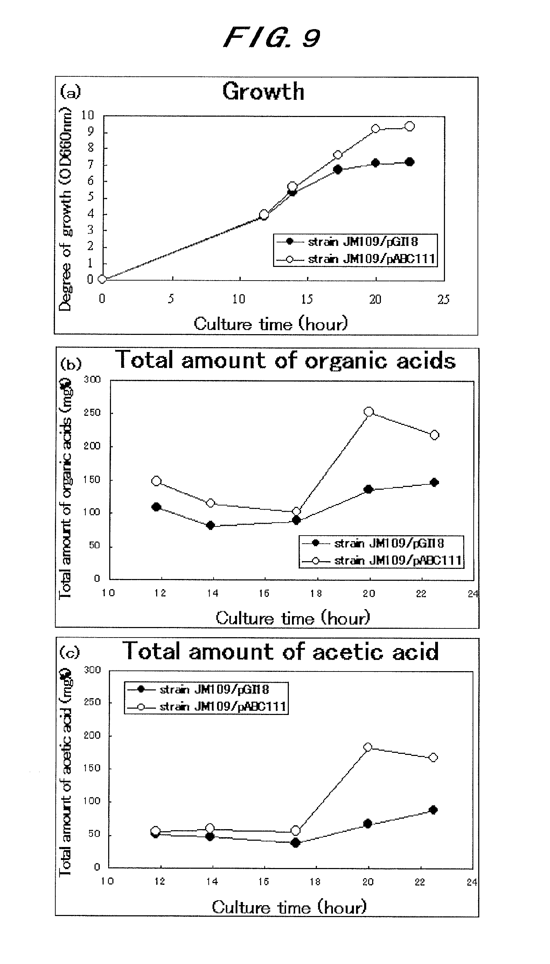

The transformant (strain JM109/pABC111) of *E*. *coli* strain JM109 comprising the plasmid pABC111 obtained in Example 2 was compared with *E*. *coli* (strain JM109/pGI18) carrying the shuttle vector pGI18, in terms of growth in the LB medium added with glucose.
Specifically, the strains were cultured aerobically (0.3 vvm) in a membrane filter-sterilized LB medium (pH 7.0) comprising 20% of glucose, and 100 µg/ml of ampicillin, using a 5-liter mini-jar fermentor (KMJ-2A manufactured by Mitsuwa Scientific Corp.), at 37°C and 500 rpm. The growth, the amount of acetic acid in broth, and the total amount of organic acids of the transformant and the non-transformant during culture or after the completion of culture were compared.
The growth was determined by measuring the absorbance (OD 660 nm) at 660 nm. The total amount of organic acids and the amount of the acetic acid in the culture were measured by Shimadzu High-Speed Liquid Chromatography Organic Acid Analysis System (Column: ShodexRSpak KC-811, Mobile Phase: 4 mM p-toluenesulfonic acid, Reaction solution: 4 mM p-toluenesulfonic acid, 80 µM EDTA, 16 mM Bis-Tris), using an appropriately diluted samples of supernatant prepared by centrifugation of the broth, and the total amount of organic acids and amount of acetic acid were calculated based on the sum of concentrations of citric acid, malic acid, succinic acid, lactic acid, acetic acid, and pyroglutamic acid, and the concentration of acetic acid, respectively.
Time courses of the growth amount of each cell, the total amount of organic acids, and the amount of the acetic acid in the culture are presented in Fig. 9. Further, the growth amount at 22 hr after start of the culture, and the concentrations of the total amount of short chain fatty acids and the amount of acetic acid in the culture broth are summarized in Table 2.

**[Table 2]**

| Strain | Growth amount (OD 660) | Total concentration of the short chain fatty acids (mg%) | Concentration of acetic acid (mg%) |
|---|---|---|---|
| strain JM109/pGI18 | 7.18 | 146.5 | 87.9 |
| strain JM109/pABC111 | 9.34 | 217.6 | 167.0 |

From the results of Fig. 9, it was confirmed that the growth amounts, the total amount of short chain fatty acids (organic acids), and the amount of acetic acid of the transformant (strain JM 109/pABC 111) were higher than those of the non-transformant (strain JM109/pGI18 strain).
Further, it was confirmed from the results of Table 2 that the growth amount at 22 hr after start of the culture, the total amount of the short chain fatty acids and the amount of acetic acid of transformant (strain JM109/pABC111) were higher than those of the non-transformant (strain JM109/pGI18).
From these findings, it was evident that *E*. *coli* obtained by introducing DNA comprising the nucleotide sequence set forth in SEQ ID NO: 1 of Sequence Listing was not adversely affected by the short chain fatty acid in the high cell-density culture, and that organic acids, as well as short chain fatty acids including acetic acid, could be produced in a large amount.

### (2) Glucose fed-batch culture

The transformant (strain JM109/pABC111) of *E*. *coli* strain JM109 comprising the plasmid pABC111 obtained in Example 2 was compared with E. *coli* (strain JM109/pGI18) carrying the shuttle vector pGI18, in terms of the growth in the fed-batch culture where glucose was fed during culture.
For the culture, a 2-liter mini-jar fermentor (KMJ-2A manufactured by Mitsuwa Scientific Corp.) and a pH controller (Digital pH controller MPH-2C manufactured by Mitsuwa Scientific Corp.) were used.

Further, as a culture medium, a medium prepared by adding 3 ml of 1 M MgSO₄ and 3ml of trace element solution (0.5 g of CaCl₂, 0.18 g of ZnSO₄·7H₂O, 0.1 g of MnSO₄·H₂O, 20.1 g of EDTA-Na₂, 16.7 g of FeCl₃·6H₂O, 0.16 g of CuSO₄·5H₂O, 0.18 g of CoCl₂·6H₂O (per liter of culture medium)) to 1 liter of Mineral medium (2.0 g of Na₂SO₄, 2.468 g of (NH₄)₂SO₄, 0.5 g of NH₄Cl, 14.6 g of K₂HPO₄, 3.6 g of NaH₂PO₄·H₂O, 1.0 g of (NH₄)₂-H-citrate, 0.05 g of Thiamin/1)) was used.
Culture was performed while controlling temperature, pH, number of stirring, and aeration at 35°C, 6.8, 700 rpm, and 0.5 1/min, respectively. The pH was adjusted by adding 29% solution of ammonia.

Cells were collected from the culture broth which had been cultured for 6 hours in 5 ml of the LB medium comprising 100 µg/ml of ampicillin, and then suspended in 5 ml of the medium. The resultant suspension was inoculated into 1 liter of the medium, and then aerobically cultured under stirring for 16.5 hours.
Thereafter, cells were aerobically cultured with stirring (aeration amount: 0.5 vvm, and stirring rate: 700 rpm) with feeding 50% solution of glucose until 26.5 hr, the feeding rate and feeding amount being increased as the fermentation proceeds, as shown in Table 3.

**[Table 3]**

| Culture time (h) | Feeding rate (ml/h) | Feeding amount (ml) |
|---|---|---|
| 0 to 16.5 | 0 | 0 |
| 16.5 to 18.5 | 10 | 20 |
| 18.5 to 20.5 | 20 | 40 |
| 20.5 to 22.5 | 30 | 60 |
| 22.5 to 24.5 | 40 | 80 |
| 24.5 to 26.5 | 50 | 100 |

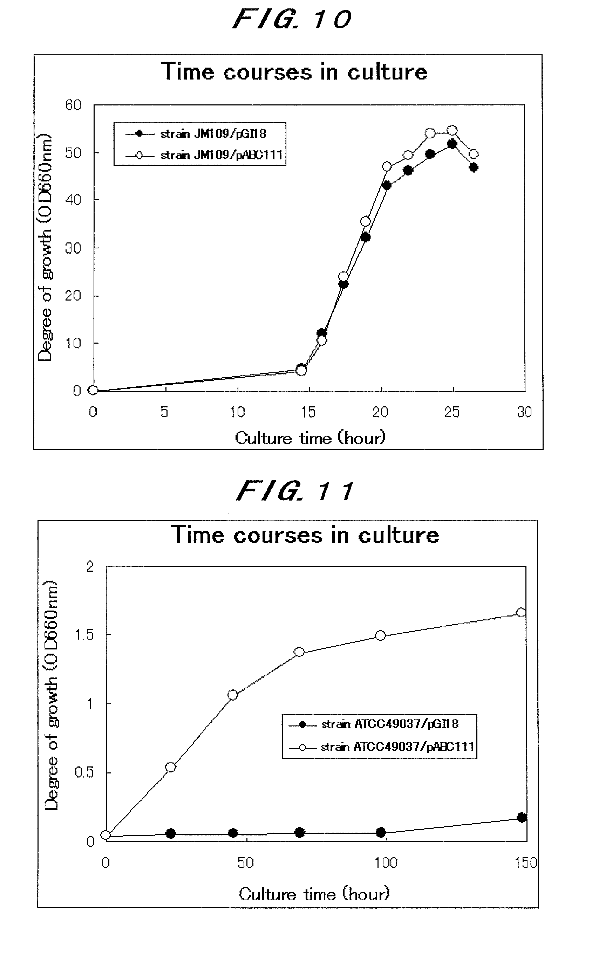

The growth was confirmed by measuring the absorbance at 660 nm (OD 660 nm) and dried cell mass.
Time course in growth (OD 660 nm) in the glucose fed-batch culture is shown in Fig. 10.
As Fig. 10 evidently shows, it was confirmed that the growth of the transformant (strain JM109/pABC111) was higher than that of the strain JM109/pGI18.
Further, at the end of the culture, the final cell mass (dried cell weight (g: DCW) and the amount of consumed glucose were measured, and based on the measured values, the cell concentration (g/l, dried cell weight per 1 L of medium), and the yield of cell mass (w/w%) were calculated. The growth rate per unit time as the average growth rate (g/h) were determined. The results for each strain were summarized in Table 4.

**[Table 4]**

| | strain JM109/pGI18 | strain JM109/pABC111 |
|---|---|---|
| Final cell mass (g) | 25.74 | 27.05 |
| Cell concentration (g/l) | 19.92 | 20.38 |
| Cell mass yield (w/w%) | 10.77 | 11.04 |
| Average growth rate (g/h) | 0.095 | 0.100 |

From the results of Table 4, it was confirmed that the cell mass, cell concentration, yield of cell mass and growth rate per unit time of transformant (strain JM109/pABC 111) were higher than those of the strain JM109/pGI18.
Accordingly, the usefulness of the high cell-density culture using the glucose fed-batch culture was confirmed.

### (Example 7) Tolerance to short chain fatty acid of Gluconacetobacter diazotrophicus transformed with DNA comprising nucleotide sequence set forth in SEQ ID NO: 1 of Sequence Listing

### (1) Preparation of transformant

*Gluconacetobacter diazotrophicus* strain ATCC49037 as one of acetic acid bacteria having no ability of acetic acid fermentation was transformed with the pABC111 constructed in Example 1 by electroporation. The transformant was selected on the YPG agar medium added with 100 µg/ml of ampicillin.
The plasmid DNA was extracted from cells of the ampicillin-resistant transformant which were cultured in the above mentioned selection medium and analyzed by the standard method, and it was confirmed that the transformant carried a plasmid comprising DNA comprising nucleotide sequence set forth in SEQ ID NO: 1 of Sequence Listing
The resulting transformant (strain ATCC49037/pABC111) was deposited at International Patent Organism Depositary, National Institute of Advanced Industrial Science and Technology (Central 6, 1-1, Higashi 1-chome, Tsukuba-shi, Ibaraki-ken, Japan) on December 14, 2004 under Deposit No. FERM BP-10186.

### (2) Tolerance to acetic acid of transformant

The resulting ampicillin-resistant transformant (strain ATCC49037/pABC111 strain), which carried the plasmid pABC111, was compared with the *Gluconacetobacter diazotrophicus* ATCC49037 strain (JM109/pGI18 strain) carrying the shuttle vector pGI18, in terms of the growth in the LB medium added with acetic acid.
Specifically, the strain was cultured with shaking (150 rpm) in the YPG medium added with 0.05% of acetic acid, and 100 µg/ml of ampicillin at 30°C. The absorbance of the broth, indicative of the growth (cell mass), was measured at 660 nm during culture and the measured values of absorbance were compared. Fig.11 shows the time course in growth (absorbance at 660 nm) during culture in each medium.

As shown in Fig. 11, it was confirmed that the transformant (strain ATCC49037/pABC111) grew in the YPG medium added with 0.05% of acetic acid, but the strain ATCC49037/pGI18 did not grow in the medium added 0.05% of acetic acid.
This demonstrates that the tolerance to acetic acid of *Gluconacetobacter diazotrophicus* can be improved by introducing DNA comprising the nucleotide sequences set forth in SEQ ID NO: 1 of Sequence Listing into *Gluconacetobacter diazotrophicus* having no ability to produce acetic acid.

### [Industrial Applicability]

According to the present invention, cells to show improved tolerance to a short chain fatty acid can be bred by conferring tolerance to a short chain fatty acid. Furthermore, when the method of breeding of the invention is applied to microbial cells, cells, whose growth is not affected by a short chain fatty acid which is produced during culture and are harmful to growth of the cell, can be bred efficiently.
The microbial cells showing tolerance to a short chain fatty acid obtained by the invention can be also applied to high cell-density culture in which short chain fatty acids are produced. Also, the cells can be used in the preparation of fermentation broths comprising short chain fatty acids at a high concentration. Particularly in the case of *Escherichia coli* to which tolerance to a short chain fatty acid is conferred, or the like, the bacterial cells show significantly improved ability to grow in medium and can efficiently accumulate the short chain fatty acid at a high concentration, thus being industrially useful.

## Claims

1. A method of breeding cells to improve tolerance to a short chain fatty acid, wherein a gene encoding a protein having a function of transporting a short chain fatty acid from the inside of cells to the outside of cells, is introduced and expressed in the cells.

2. The method of breeding cells according to claim 1, wherein the gene encoding the protein having a function of transporting a short chain fatty acid from the inside of cells to the outside of cells is a DNA represented by the following (a) or (b):
(a) a DNA that comprises a nucleotide sequence consisting of nucleotide numbers 301 to 2073 in the nucleotide sequence set forth in SEQ ID NO: 1 of the Sequence Listing; or
(b) a DNA that comprises a nucleotide sequence which hybridizes with a probe comprising a nucleotide sequence consisting of nucleotide numbers 301 to 2073 in the nucleotide sequence set forth in SEQ ID NO: 1 of the Sequence Listing or comprising at least a part of said nucleotide sequence under stringent conditions, and that encodes a protein which is capable of enhancing tolerance to acetic acid.

3. The method of breeding cells according to claim 1, wherein the gene encoding the protein having a function of transporting a short chain fatty acid from the inside of cells to the outside of cells is a DNA represented by the following (a) or (b):
(a) a DNA that comprises a nucleotide sequence consisting of nucleotide numbers 331 to 2154 in the nucleotide sequence set forth in SEQ ID NO: 3 of the Sequence Listing; or
(b) a DNA that comprises a nucleotide sequence which hybridizes with a probe comprising a nucleotide sequence consisting of nucleotide numbers 331 to 2154 in the nucleotide sequence set forth in SEQ ID NO: 3 of the Sequence Listing or comprising at least a part of said nucleotide sequence under stringent conditions, and that encodes a protein which is capable of enhancing tolerance to acetic acid.

4. The method of breeding cells according to claim 1, wherein the gene encoding the protein having a function of transporting a short chain fatty acid from the inside of cells to the outside of cells is a DNA represented by the following (a), (b), (c) or (d):
(a) a DNA that comprises a nucleotide sequence consisting of nucleotide numbers 1724 to 2500 and a nucleotide sequence consisting of nucleotide numbers 1724 to 2500 in the nucleotide sequence set forth in SEQ ID NO:5 of in the Sequence Listing;
(b) a DNA that comprises both a nucleotide sequence which hybridizes with a probe comprising a nucleotide sequence consisting of nucleotide numbers 1002 to 1724 in the nucleotide sequence set forth in SEQ ID NO:5 of the Sequence Listing or comprising at least a part thereof under stringent conditions, and a nucleotide sequence consisting of nucleotide numbers 1724 to 2500 in the nucleotide sequence set forth in SEQ ID NO:5 of the Sequence Listing, and that encodes a protein complex which is capable of enhancing tolerance to acetic acid;
(c) a DNA that comprises both a nucleotide sequence consisting of nucleotide numbers 1002 to 1724 in the nucleotide sequence set forth in SEQ ID NO:5 of the Sequence Listing, and a nucleotide sequence which hybridizes with a probe comprising a nucleotide sequence consisting of nucleotide numbers 1724 to 2500 in the nucleotide sequence set forth in SEQ ID NO:5 of the Sequence Listing or comprising at least a part thereof under stringent conditions, and that encodes a protein complex which is capable of enhancing tolerance to acetic acid; or
(d) a DNA that comprises both a nucleotide sequence which hybridizes with a probe comprising a nucleotide sequence consisting of nucleotide numbers 1002 to 1724 in the nucleotide sequence set forth in SEQ ID NO:5 of the Sequence Listing or comprising at least a part thereof under stringent conditions and a nucleotide sequence which hybridizes with a probe comprising a nucleotide sequence consisting of nucleotide numbers 1724 to 2500 in the nucleotide sequence set forth in SEQ ID NO:5 of the Sequence Listing or comprising at least a part thereof under stringent conditions, and that encodes a protein complex which is capable of enhancing tolerance to acetic acid.

5. The method of breeding cells according to claim 1, wherein the gene encoding the protein having a function of transporting a short chain fatty acid from the inside of cells to the outside of cells, is a DNA represented by the following (a) or (b):
(a) a DNA that comprises a nucleotide sequence consisting of nucleotide numbers 249 to 1025 in the nucleotide sequence set forth in SEQ ID NO: 8 of the Sequence Listing; or
(b) a DNA that comprises a nucleotide sequence which hybridizes with a probe comprising a nucleotide sequence consisting of nucleotide numbers 249 to 1025 in the nucleotide sequence set forth in SEQ ID NO: 8 of the Sequence Listing or comprising at least a part of said nucleotide sequence under stringent conditions, and that encodes a protein which is capable of enhancing tolerance to acetic acid.

6. The method of breeding cells according to any one of claims 1 to 5, wherein the short chain fatty acid is formic acid, acetic acid, propionic acid, butyric acid, isobutyric acid, or valeric acid.

7. Cells bred by the method according to any one of claims 1 to 5, wherein the cells are microbial cells.

8. The cells according to claim 7, wherein the microbial cells are cells of acetic acid bacteria, bacteria belonging to the genus *Escherichia,* or genus *Bacillus.*

9. A method for high cell-density culture that uses the cells according to claim 8.

10. The method for high cell-density culture according to claim 9, wherein the cells are cultured in the presence of a short chain fatty acid.

11. A method of preparing a fermentation broth comprising a short chain fatty acid, wherein the cells according to claim 8 are cultured under the conditions such that the cells produce a short chain fatty acid.


SEQUENCE LISTING

<110>  Mitsukan Group Corporation

<120>  METHOD OF BREEDING CELLS TO IMPROVE TOLERANCE TO SHORT CHAIN FATTY ACIDS

<130>  A-367

<160>  18

<170>  PatentIn version 3.1

<210>  1
<211>  2414
<212>  DNA
<213>  Acetobacter aceti

<400>  1
gctcgcgtac ccgggcgwtc ctctagagtc atcaaccttg gcccaggtgg ggatggcata      60

caggcggtgg cggaaattac ctcatctttc agtgtgccgg ttatatacgt aacggcgtat     120

ccagaacgtt tgctaactgg ggaaaccatg gagcccagtt ttgttattac caagccgttt     180

gaccccctta cccttgctgt tgcaacgtat caggcagtaa gcagcgcacg cacacaggcc     240

gtataagcaa aaaagcggcc tccatttcca gttctacaaa acggattatt tttttccagc     300

atggcgcatc ctccccttct tcatcttcag gacattactc tttcattagg agggaacccg     360

ctgctggatg gcgccggttt tgccgttggg cgtggtgagc gcctctgcct tgtggggcga     420

aacggttcgg gaaagtccac cctgctcaaa attgctgcgg gtgttattca gccagattcg     480

gggtctgtgt ttgtccagcc cggtgcttcc ctgcgctatc tgccgcagga gccggattta     540

agcgcttatg ccacaacggc ggattacgtt gtgggccaga ttggagaccc ggatatggca     600

tggcgcgcca cgccattgct ggatgctctg ggcctgacag gtagggaaag cacgcaaaat     660

ctttcaggcg gtgaaggtcg gcgttgtgct attgctggtg tattggcggc ggcccccgat     720

gtgctgctgc tggatgagcc caccaaccat ctggatatgc ctaccattga atggttggag     780

cgtgaactgc tgagccttgg cgccatggta attatcagcc atgataggcg gctgctttcc     840

accctttcac gttctgttgt gtggctggat cggggtgtaa cccgcaggct tgatgaagga     900

tttggaaggt ttgaagcctg gcgagaggag gttctggaac aggaagagcg tgatgcgcat 960

aaactggacc ggaaaatcgc gcgggaagaa gactggatgc gttatggcgt aacggcgcgc 1020

cgcaaacgca atgtacgccg tgtgcgggaa ctagcagatt tgcgcacagc ccgtaaggag 1080

gccattcggg cacccggcac ccttaccttg aacacgcagc tgcggccaca tcgcaagctg 1140

gtggctgtgg ccgaagatat tagtaaggca tggggtgaaa agcaggttgt tcgccatttg 1200

gacctgcgca ttttacgtgg agaccggctt ggtattgtgg gggccaatgg tgcaggcaaa 1260

accacattgt tgcggatgct aacagggctg gaccaacccg atagtggcac aatctcactt 1320

ggtccttccc ttaatatggt cacgctggat cagcagcgac gtaccctgaa cccggaacgc 1380

acactagccg ataccttgac agaaggcgga ggcgatatgg tgcaggttgg cacggaaaag 1440

cgccacgttg tggggtatat gaaagacttt ctgtttcggc cagaacaggc acgcacaccc 1500

gtaagtgccc tttctggcgg ggagcgaggg cggttaatgc tggcatgcgc attggccaag 1560

ccctccaacc tgctggtgct ggatgaaccc accaatgatc tggatctgga aacactggat 1620

attttgcaag acatgctcgc cagttgtgaa ggcacagtgc tgcttgtaag ccatgatcgt 1680

gattttctgg atcgggttgc aacatccgtc ttggcgacag agggagatgg caactggata 1740

gaatatgctg gcggatacag tgacatgctg gctcagcggc accagaaacc gttgacaacg 1800

gcctctgtgg tggaaaacga acccacaaaa cccaaagaga caactgctgc gcgtggcccg 1860

accaaaaagc tgagttataa ggaccagttt gcgctggata atctgcccaa ggaaatggaa 1920

aagctggaag cacaggctgc caactgcgtg aaaaactggc agatccagat ttatatggaa 1980

aaaaccccgc gcagtttgag aaactttcgg ctgatttaca gaagctcgaa acaaagctgg 2040

cagaatctga agaacgctgg ctggaactgg aaatgaagcg agaagcccta caggccaact 2100

aaggcaacgc tatttttcgg tgaaccgcac tcttgcaggc gggtgggtgc aatgcctatg 2160

ttttggcatg ctctgtttta ctggttctct ttataagcgc acccttcctg ctggcagtct 2220

ggcattgctt gcctttctga gcgtggcaca cattgcattc gcgcaggata dacccgccgc 2280

tgcagtctcc ctatagtgag tcgtattacg cgttctaacg aatccatatg actwtgtaga 2340

ccctctagag tcgacctgca ggcatgcaag cttyccctat agtgagtcgt attagagctt 2400

ggcgtaatgc atga 2414

<210> 2
<211> 591
<212> PRT
<213> Acetobacter aceti

<400> 2

Met Ala His Pro Pro Leu Leu His Leu Gln Asp Ile Thr Leu Ser Leu
1               5                   10                  15

Gly Gly Asn Pro Leu Leu Asp Gly Ala Gly Phe Ala Val Gly Arg Gly
            20                  25                  30

Glu Arg Leu Cys Leu Val Gly Arg Asn Gly Ser Gly Lys Ser Thr Leu
        35                  40                  45

Leu Lys Ile Ala Ala Gly Val Ile Gln Pro Asp Ser Gly Ser Val Phe
    50                  55                  60

Val Gln Pro Gly Ala Ser Leu Arg Tyr Leu Pro Gln Glu Pro Asp Leu
65                  70                  75                  80

Ser Ala Tyr Ala Thr Thr Ala Asp Tyr Val Val Gly Gln Ile Gly Asp
                85                  90                  95

Pro Asp Met Ala Trp Arg Ala Thr Pro Leu Leu Asp Ala Leu Gly Leu
            100                 105                 110

Thr Gly Arg Glu Ser Thr Gln Asn Leu Ser Gly Gly Glu Gly Arg Arg
        115                 120                 125

Cys Ala Ile Ala Gly Val Leu Ala Ala Ala Pro Asp Val Leu Leu Leu
    130                 135                 140

Asp Glu Pro Thr Asn His Leu Asp Met Pro Thr Ile Glu Trp Leu Glu
145                 150                 155                 160

Arg Glu Leu Leu Ser Leu Gly Ala Met Val Ile Ile Ser His Asp Arg
                165                 170                 175

Arg Leu Leu Ser Thr Leu Ser Arg Ser Val Val Trp Leu Asp Arg Gly
            180                 185                 190

Val Thr Arg Arg Leu Asp Glu Gly Phe Gly Arg Phe Glu Ala Trp Arg
        195                 200                 205

Glu Glu Val Leu Glu Gln Glu Glu Arg Asp Ala His Lys Leu Asp Arg
    210                 215                 220

Lys Ile Ala Arg Glu Glu Asp Trp Met Arg Tyr Gly Val Thr Ala Arg
225                 230                 235                 240

Arg Lys Arg Asn Val Arg Arg Val Arg Glu Leu Ala Asp Leu Arg Thr
                245                 250                 255

Ala Arg Lys Glu Ala Ile Arg Ala Pro Gly Thr Leu Thr Leu Asn Thr
            260                 265                 270

Gln Leu Arg Pro His Arg Lys Leu Val Ala Val Ala Glu Asp Ile Ser
        275                 280                 285

Lys Ala Trp Gly Glu Lys Gln Val Val Arg His Leu Asp Leu Arg Ile
    290                 295                 300

Leu Arg Gly Asp Arg Leu Gly Ile Val Gly Ala Asn Gly Ala Gly Lys
305                 310                 315                 320

Thr Thr Leu Leu Arg Met Leu Thr Gly Leu Asp Gln Pro Asp Ser Gly
                325                 330                 335

Thr Ile Ser Leu Gly Pro Ser Leu Asn Met Val Thr Leu Asp Gln Gln
            340                 345                 350

Arg Arg Thr Leu Asn Pro Glu Arg Thr Leu Ala Asp Thr Leu Thr Glu
        355                 360                 365

Gly Gly Gly Asp Met Val Gln Val Gly Thr Glu Lys Arg His Val Val
    370                 375                 380

Gly Tyr Met Lys Asp Phe Leu Phe Arg Pro Glu Gln Ala Arg Thr Pro
385                 390                 395                 400

Val Ser Ala Leu Ser Gly Gly Glu Arg Gly Arg Leu Met Leu Ala Cys
                405                 410                 415

Ala Leu Ala Lys Pro Ser Asn Leu Leu Val Leu Asp Glu Pro Thr Asn
            420                 425                 430

Asp Leu Asp Leu Glu Thr Leu Asp Ile Leu Gln Asp Met Leu Ala Ser
        435                 440                 445

Cys Glu Gly Thr Val Leu Leu Val Ser His Asp Arg Asp Phe Leu Asp
    450                 455                 460

Arg Val Ala Thr Ser Val Leu Ala Thr Glu Gly Asp Gly Asn Trp Ile
465                 470                 475                 480

Glu Tyr Ala Gly Gly Tyr Ser Asp Met Leu Ala Gln Arg His Gln Lys
                485                 490                 495

Pro Leu Thr Thr Ala Ser Val Val Glu Asn Glu Pro Thr Lys Pro Lys
            500                 505                 510

Glu Thr Thr Ala Ala Arg Gly Pro Thr Lys Lys Leu Ser Tyr Lys Asp
        515                 520                 525

Gln Phe Ala Leu Asp Asn Leu Pro Lys Glu Met Glu Lys Leu Glu Ala
    530                 535                 540

Gln Ala Ala Asn Cys Val Lys Asn Trp Gln Ile Gln Ile Tyr Met Glu
545                 550                 555                 560

Lys Thr Pro Arg Ser Leu Arg Asn Phe Arg Leu Ile Tyr Arg Ser Ser
                565                 570                 575

Lys Gln Ser Trp Gln Asn Leu Lys Asn Ala Gly Trp Asn Trp Lys
            580                 585                 590

<210>  3
<211>  2160
<212>  DNA
<213>  Gluconacetobacter entanii

<400>  3
atcttgtggc caaggaattc atcgcagccc aggatcctga aaatcccggc gtgctgatcc      60

tctcccgcct tgccggggcg gcaaagcagc ttgaggccgc cctgctggtc aacccgctgg     120

atcatgacgg catggccgat gcgctggaac gcgcgctggc catgtcgccc gaggaacggc     180

gcgaacgctg gcaggcatgc tggaacagca ttgccaaccg tacggccctt ggctgggggc     240

tgtcgttcct gaacatcctt gaaaacgcga agcggcgtta agccccacac cagccttgcg     300

cacggggtgg ttgagaatac ataagtgggc atggcctcac ctccccttct tctccttcag     360

gatatcaccc tgacccttgg cggcgcgccg ctgctcaatg gcgcgggctt cggcgttggc     420

cctggcgagc gcgtctgcct tgtcgggcgc aatggctgtg gcaagtccac cctgctgcgc     480

atcgcggcgg gtgagataca ggccgatgac ggcaccgttt ttgtccagcc cggcaccacc     540

gtgcgctacc tgccgcagga acccgacctg tcgggctttg acaccacgct ggattacgtc     600

cgcgcgggca tggggccggg cgacccggaa taccgcgccg aactgctgct gaccgaactg     660

gggctgaacg gcacggaaga cccggccacc ctgtcgggcg gggaagcgcg gcgctgcgcg     720

ctggcccgcg cccttgcgcc cgaacccgac ctgcttttgc tggacgaacc caccaaccac     780

ctggacatgc ccaccattga atggctggaa cgtgaactgc tgtcgctgtc atcggccatg 840

gtcatcataa gccatgaccg caggctgctg gaaacgctgt cgcgttcggt cgtgtggctg 900

gaccggggtg tcacccgcag gctggatcag ggcttcgccc ggttcgagac atggcgcgag 960

gaagtgctgg agcaggaaga gcgcgacagc cacaagctgg accgccagat cgcgcgtgag 1020

gaagactgga tgcgttacgg cgtgaccgcg cggcgcaagc gcaatgtccg ccgcgtggct 1080

gaactggccg aactgcgcaa tacccgtcgc accgccataa ggcagcccgg cggcctgaag 1140

atggaagccc gcgaaagcga cctgtcgggc aagctggttg cggtggcaga agatatgtca 1200

cgcgcctatg accctgccca cccggtggtc agccatctgg acctgcgtgt cctgcgcggg 1260

gaccggctgg ggatcgtggg ggccaatggc gcgggcaaga gcaccctgct gcgcctgctg 1320

acgggactgg acaggccgga ttccggcacc atcaatatcg gcagcgcgct caatgtcgtc 1380

acactggacc agcagcgccg ctcgcttgat cccgacacca cgctggcgga tacgctgacg 1440

ggcggcggcg gggacatggt gcaggttggc aatgagaaac gccatgtcat cggctacatg 1500

aaggacttcc tgttccgccc cgaacaggcg cgtaccccgg tgggcgtgct gtcggggggg 1560

gagcgctggc ggctcatgct ggcctgcgcg ctggcgcggc cgtccaacct gctggtgctg 1620

gacgagccga ccaacgacct tgaccttgaa acgctcgacc tgctgcagga catgctggcc 1680

agctattccg gcacggtgct gctggtcagc catgaccgtg acttcctcga ccgggtcgcc 1740

tcctccatcc tgatggcgga aggcggcgga aagtgggtgg aatatgccgg tggctacagc 1800

gacatgctgg cccagcggca ggacgccaca ctggccgccc gcccccggca ggaccgcgcg 1860

gaaaccacac cggccagaac cgatgtgacc ccgtcctcct ccccccggca gcccgcgcgc 1920

aagatgtcgt acaaggacaa gcacgcgctg gaacagctac ccaagcagat ggcggcgctg 1980

gagacggaaa tcgagcgcct gcgcgccatc ctgtccgacg ggggcctgta tgcgcgcgac 2040

cccgccacct ttacggccgc caccacggcg ctggaaaagg cagaggccga cctgacggcg 2100

gcggaagaac ggtggctgga actcgaaatg ctgcgcgaga cgcttcagtc ttcctgaacg 2160

<210> 4

<211>  608
<212>  PRT
<213>  Gluconacetobacter entanii

<400>  4

Met Ala Ser Pro Pro Leu Leu Leu Leu Gln Asp Ile Thr Leu Thr Leu
1               5                   10                  15

Gly Gly Ala Pro Leu Leu Asn Gly Ala Gly Phe Gly Val Gly Pro Gly
            20                  25                  30

Glu Arg Val Cys Leu Val Gly Arg Asn Gly Cys Gly Lys Ser Thr Leu
        35                  40                  45

Leu Arg Ile Ala Ala Gly Glu Ile Gln Ala Asp Asp Gly Thr Val Phe
    50                  55                  60

Val Gln Pro Gly Thr Thr Val Arg Tyr Leu Pro Gln Glu Pro Asp Leu
65                  70                  75                  80

Ser Gly Phe Asp Thr Thr Leu Asp Tyr Val Arg Ala Gly Met Gly Pro
                85                  90                  95

Gly Asp Pro Glu Tyr Arg Ala Glu Leu Leu Leu Thr Glu Leu Gly Leu
            100                 105                 110

Asn Gly Thr Glu Asp Pro Ala Thr Leu Ser Gly Gly Glu Ala Arg Arg
        115                 120                 125

Cys Ala Leu Ala Arg Ala Leu Ala Pro Glu Pro Asp Leu Leu Leu Leu
    130                 135                 140

Asp Glu Pro Thr Asn His Leu Asp Met Pro Thr Ile Glu Trp Leu Glu
145                 150                 155                 160

Arg Glu Leu Leu Ser Leu Ser Ser Ala Met Val Ile Ile Ser His Asp
                165                 170                 175

Arg Arg Leu Leu Glu Thr Leu Ser Arg Ser Val Val Trp Leu Asp Arg
            180                 185                 190

Gly Val Thr Arg Arg Leu Asp Gln Gly Phe Ala Arg Phe Glu Thr Trp
        195                 200                 205

Arg Glu Glu Val Leu Glu Gln Glu Glu Arg Asp Ser His Lys Leu Asp
    210                 215                 220

Arg Gln Ile Ala Arg Glu Glu Asp Trp Met Arg Tyr Gly Val Thr Ala
225                 230                 235                 240

Arg Arg Lys Arg Asn Val Arg Arg Val Ala Glu Leu Ala Glu Leu Arg
                245                 250                 255

Asn Thr Arg Arg Thr Ala Ile Arg Gln Pro Gly Gly Leu Lys Met Glu
            260                 265                 270

Ala Arg Glu Ser Asp Leu Ser Gly Lys Leu Val Ala Val Ala Glu Asp
        275                 280                 285

Met Ser Arg Ala Tyr Asp Pro Ala His Pro Val Val Ser His Leu Asp
    290                 295                 300

Leu Arg Val Leu Arg Gly Asp Arg Leu Gly Ile Val Gly Ala Asn Gly
305                 310                 315                 320

Ala Gly Lys Ser Thr Leu Leu Arg Leu Leu Thr Gly Leu Asp Arg Pro
                325                 330                 335

Asp Ser Gly Thr Ile Asn Ile Gly Ser Ala Leu Asn Val Val Thr Leu
            340                 345                 350

Asp Gln Gln Arg Arg Ser Leu Asp Pro Asp Thr Thr Leu Ala Asp Thr
        355                 360                 365

Leu Thr Gly Gly Gly Gly Asp Met Val Gln Val Gly Asn Glu Lys Arg
    370                 375                 380

His Val Ile Gly Tyr Met Lys Asp Phe Leu Phe Arg Pro Glu Gln Ala
385                 390                 395                 400

Arg Thr Pro Val Gly Val Leu Ser Gly Gly Glu Arg Trp Arg Leu Met
                405                 410                 415

Leu Ala Cys Ala Leu Ala Arg Pro Ser Asn Leu Leu Val Leu Asp Glu
            420                 425                 430

Pro Thr Asn Asp Leu Asp Leu Glu Thr Leu Asp Leu Leu Gln Asp Met
        435                 440                 445

Leu Ala Ser Tyr Ser Gly Thr Val Leu Leu Val Ser His Asp Arg Asp
    450                 455                 460

Phe Leu Asp Arg Val Ala Ser Ser Ile Leu Met Ala Glu Gly Gly Gly
465                 470                 475                 480

Lys Trp Val Glu Tyr Ala Gly Gly Tyr Ser Asp Met Leu Ala Gln Arg
                485                 490                 495

Gln Asp Ala Thr Leu Ala Ala Arg Pro Arg Gln Asp Arg Ala Glu Thr
            500                 505                 510

Thr Pro Ala Arg Thr Asp Val Thr Pro Ser Ser Ser Pro Arg Gln Pro
        515                 520                 525

Ala Arg Lys Met Ser Tyr Lys Asp Lys His Ala Leu Glu Gln Leu Pro
    530                 535                 540

Lys Gln Met Ala Ala Leu Glu Thr Glu Ile Glu Arg Leu Arg Ala Ile
545                 550                 555                 560

Leu Ser Asp Gly Gly Leu Tyr Ala Arg Asp Pro Ala Thr Phe Thr Ala
                565                 570                 575

Ala Thr Thr Ala Leu Glu Lys Ala Glu Ala Asp Leu Thr Ala Ala Glu
            580                 585                 590

Glu Arg Trp Leu Glu Leu Glu Met Leu Arg Glu Thr Leu Gln Ser Ser
        595                 600                 605

<210>  5
<211>  3188
<212>  DNA
<213>  Gluconacetobacter entanii

<400>  5
ccgggcgcgt ggggacgaag tccggcccgg actgatacgc gcgatcgcgg atacgctggc      60

gcgcgtgttg cccgcatggc ccggcacggg cggcaacccg tccctgccgc gtgggcagat     120

ccatgccgac ctgtttcccg acaatgtctt tttccgtgac gggaaactgt cgggcatcat     180

tgatttctat ttcgcctgca ccgactggta cgcctacgac ctggccatta ccgtcaatgc     240

atggtgtttt gatgacatgg gccgtttcgt gcccacccgt gcgcgcgcca tggtggaagc     300

ctaccgccat gtccgcccgc tggaggatgc ggaggacgcg gccctggcca cccttgccac     360

gggtgcggcc atccgtttca cgctgacccg cctgtatgac tggatcaata cgcccccgga     420

tgcgctggtg acgcgcaagg acccgctgga ctatctggcg cgcatggaat tcttcgcgtc     480

ccgcatggat gaaggtttcc tgtgatggac gaggacatgg cccccgtgga aaacgtggca     540

cccgccaccg acatggtgga aatctggacc gatggcgggt gcaagcccaa tcccggcccc     600

ggtggctggg gcgcattgct gtgctgtcgc gggcaggagc gtgaactgtc gggtggcgag     660

gcggaaacca caaacaaccg catggaactg accgccgcgg ccgaggcgct ggaggcgctg     720

aaacgtccct gccgtgtcgt gctgcacacc gacagcgaat atgtgcgcaa tggcatcacg     780

cggtggagca cgggctgggt gcggcgcaaa tggcgcaatg catccggtga tccggtggcg     840

aacatggatc tgtggcggcg gctgctggat gtcagcgcga agcacgagat cgagtggaaa 900

tgggtccgtg gccattccgg cgacgtgaat aacgaacgcg tggaccagat ggccacttcg 960

gcgcgtgacg cgctgggcat tccctatccc aagcgtggaa aatgatgcgc gcgccccctg 1020

ccatccgtct ggaaggggtg ggctggattt tcggggccgc gccgcttttc cgtaatctgg 1080

acctgtgcat cggggccggg accatgaccg tgctgctggg tgccagcggt gttggcaaga 1140

catcgctgct gcgcatgctg ggcggcttgg tcgcccctga ttactggcgt gtcgtggccg 1200

gggacgggct gccccttgcc ggacgggtcg catggatggg gcagcaggat ctgctgctgc 1260

catgggcgac tgccatggac aatgtaatgc tgggcgcgcg cctgcgtggc gaccggctgg 1320

accgcgacag ggcagggtgc ctgctggatt gtgtcgggct gtcatcgcac gcggcggccc 1380

ttccggccac gctgtcgggg ggcatgcggc agcgtgtggc gctggcgctg gtattgtatg 1440

aagaccgtcc ggtcgtgctg atggatgaac ctttttccgc actggacagt gtgacacggg 1500

cacgcatgca ggatctggcc ggccggatgc tggcgggccg gaccgtggtg ctgattaccc 1560

atgacccgct ggaagcctgc cgtctggcgg atcacatgct gctcatggcc gggcatcccg 1620

cacggctgga cggcattgcc gtcccggcgg ggaccgtgcc gcgcgcggtg gatgacgcgg 1680

gcgtgcttgc ggcgcagtcc gcgctgctgc ggcggatgat gcaatgaccg gcaaggccac 1740

ggcacggggc atgcgcctgc tgcgcccgct tgtcacgctg gccggtctgg tggccgtgtg 1800

ggcgcgcttg cgcgctgggg ggcatgtgcc gccctatatg ctgcccggcc ccgatgcggt 1860

ggcgcgtgcg ctgtggacgc agcgcgcgca actggcccca gccgccctga ccacgctgga 1920

ggagacggtt ctgggccttg tgctgggaat cggggcgggg ggcgcgctgg ccatcggcat 1980

ggcggtctgc gcgccgctgc ggcggtgggt catgcccatg gtgctgctca gccaggcggt 2040

gccggttttc gcgctggcgc cgctgctggt gctgtggttc ggattcggca tggcgtccaa 2100

ggtggtcatg gcggtgctgg tcattttctt tcccgtcacg tcggcgcttg gcgatggcct 2160

gcgccagacc gagccggggt ggatggacct tgcccgcacc atgggggcca cgcggtggcg 2220

ggtgctggtg catgtgcgcc tgcccgcggc cctgccgtcc tttgcctcgg gcgtgcgcat 2280

ggccaccgcc atcgccccga tcggggccgt cgtgggggag tgggtcgggg cgtcgtccgg 2340

cctctggttc ctgatgcaga cggccaatac ccgattccag acggatctca tgtttgccgc 2400

tctcgcggtg ctggcggtca tgaccgtgct gctgtggtgg ggcgtggacc ggttgctggc 2460

gcgtgcgctg tactggctgc cccggcatgc tgatgttgac tgaactgttg taacacgtca 2520

ccaccaccgc taatcgcgca acccaatgca ggatggatgc ccaatgacac gccccatcat 2580

tcttgacctg acacccggcg cgcagggaat ggcgaccctg ctggcggcgc tgcaggtgcc 2640

tgaccacgtg cgcccggtgc tggtgctgtt cagcggcagg gccgccgaag tcgaagtggc 2700

gctgacccat gcgcgtgacc tgctgcaccg gtacgggctg gatgatgtct ccgtgtgcgc 2760

gggctgtccc ggcccgatgg tgcaggccgg ggacgtgggg catgacctgc ccgcggggca 2820

ggatgggctg ggcgcgctgc atctggtgcg ggcggtgcgg gcctgttcgg cggacagcgt 2880

gagcatatgc tgttccggcc cgctgaccac gctggccgtg gccctggtgc aggcgcccga 2940

catggcttcg cacctgcatg gggtgattgt gaatggcggc gcgtttttcg tgcatggcga 3000

tgccaccagc gtggccgaac gcaatatcgc ggccgacccg gaagccgcgg ctgtggtgct 3060

ggcggcgggc gtgccggtga ccattgtgcc gctggactgc gccgcgcgcc tgacggcgga 3120

tgcggtgtgg atggaacagc ttgagccgat gggccgcgtc ccggcttccg tcgcgggcag 3180

ggtgcatg 3188

<210> 6
<211> 241
<212> PRT
<213> Gluconacetobacter entanii

<400> 6

Met Met Arg Ala Pro Pro Ala Ile Arg Leu Glu Gly Val Gly Leu Asp
1               5                   10                  15

Phe Gly Ala Ala Pro Leu Phe Arg Asn Leu Asp Leu Cys Ile Gly Ala
            20                  25                  30

Gly Thr Met Thr Val Leu Leu Gly Ala Ser Gly Val Gly Lys Thr Ser
        35                  40                  45

Leu Leu Arg Met Leu Gly Gly Leu Val Ala Pro Asp Tyr Trp Arg Val
    50                  55                  60

Val Ala Gly Asp Gly Leu Pro Leu Ala Gly Arg Val Ala Trp Met Gly
65                  70                  75                  80

Gln Gln Asp Leu Leu Leu Pro Trp Ala Thr Ala Met Asp Asn Val Met
                85                  90                  95

Leu Gly Ala Arg Leu Arg Gly Asp Arg Leu Asp Arg Asp Arg Ala Gly
            100                 105                 110

Cys Leu Leu Asp Cys Val Gly Leu Ser Ser His Ala Ala Ala Leu Pro
        115                 120                 125

Ala Thr Leu Ser Gly Gly Met Arg Gln Arg Val Ala Leu Ala Leu Val
    130                 135                 140

Leu Tyr Glu Asp Arg Pro Val Val Leu Met Asp Glu Pro Phe Ser Ala
145                 150                 155                 160

Leu Asp Ser Val Thr Arg Ala Arg Met Gln Asp Leu Ala Gly Arg Met
                165                 170                 175

Leu Ala Gly Arg Thr Val Val Leu Ile Thr His Asp Pro Leu Glu Ala
            180                 185                 190

Cys Arg Leu Ala Asp His Met Leu Leu Met Ala Gly His Pro Ala Arg
        195                 200                 205

Leu Asp Gly Ile Ala Val Pro Ala Gly Thr Val Pro Arg Ala Val Asp
    210                 215                 220

Asp Ala Gly Val Leu Ala Ala Gln Ser Ala Leu Leu Arg Arg Met Met
225                 230                 235                 240

Gln

<210> 7
<211> 259
<212> PRT
<213> Gluconacetobacter entanii

<400> 7

Met Thr Gly Lys Ala Thr Ala Arg Gly Met Arg Leu Leu Arg Pro Leu
1               5                   10                  15

Val Thr Leu Ala Gly Leu Val Ala Val Trp Gly Ala Leu Ala Arg Trp
            20                  25                  30

Gly His Val Pro Pro Tyr Met Leu Pro Gly Pro Asp Ala Val Ala Arg
        35                  40                  45

Ala Leu Trp Thr Gln Arg Ala Gln Leu Ala Pro Ala Ala Leu Thr Thr
    50                  55                  60

Leu Glu Glu Thr Val Leu Gly Leu Val Leu Gly Ile Gly Ala Gly Gly
65                  70                  75                  80

Ala Leu Ala Ile Gly Met Ala Val Cys Ala Pro Leu Arg Arg Trp Val
                85                  90                  95

Met Pro Met Val Leu Leu Ser Gln Ala Val Pro Val Phe Ala Leu Ala
            100                 105                 110

Pro Leu Leu Val Leu Trp Phe Gly Phe Gly Met Ala Ser Lys Val Val
        115                 120                 125

Met Ala Val Leu Val Ile Phe Phe Pro Val Thr Ser Ala Leu Gly Asp

130                 135                 140

Gly Leu Arg Gln Thr Glu Pro Gly Trp Met Asp Leu Ala Arg Thr Met
145                 150                 155                 160

Gly Ala Thr Arg Trp Arg Val Leu Val His Val Arg Leu Pro Ala Ala
                165                 170                 175

Leu Pro Ser Phe Ala Ser Gly Val Arg Met Ala Thr Ala Ile Ala Pro
            180                 185                 190

Ile Gly Ala Val Val Gly Glu Trp Val Gly Ala Ser Ser Gly Leu Trp
        195                 200                 205

Phe Leu Met Gln Thr Ala Asn Thr Arg Phe Gln Thr Asp Leu Met Phe
    210                 215                 220

Ala Ala Leu Ala Val Leu Ala Val Met Thr Val Leu Leu Trp Trp Gly
225                 230                 235                 240

Val Asp Arg Leu Leu Ala Arg Ala Leu Tyr Trp Leu Pro Arg His Ala
                245                 250                 255

Asp Val Asp

<210>  8
<211>  1260
<212>  DNA
<213>  Gluconacetobacter entanii

<400>  8
ggccatatcg tgatccgcac ccgcacggag accataaccg gggaccgggg cgtatatgtg      60

cccgatacgg gcattgcccg tctggttggc aacgtgcata tcacccgtgg ggaaaatcag     120

gtcagcggca cgtcggccat cgtgaacatg cataccgata tcgcgacgct gaccgataac     180

cccggctcac gtgtcagcgg cctggtcatt ccgaaccagg ctggcaaagg tgacaaggga     240

tcagcaagat gaacacgaca tccccggcgg accaggccac caccgaacag ccgatccccg 300

cagccgaagc ggggctgatc gccagcggca tcggcaagag ctacaagaaa cggcaggtcg 360

tgcgcgatgt ctcgctgcag gtccagcgtg gcgaggccgt ggccctgctg ggcccgaacg 420

gcgcgggcaa gaccaccagc ttctacatga tcgtgggact ggtgcggccc gacatgggca 480

cgatcacgct ggatggcgcg gatatcaccc agttgcccat gtaccgccgc gcccgcatgg 540

gcattggcta cctgccgcag gaatcaagca ttttccgtgg cctgaatgtt gaacagaaca 600

tcatggcggc gctggagatc gtggaacccg accgggaccg gcggcagacg atgcttgacg 660

ggctgctggg ggaattcggc attacccggc tgcggcattc atcctccctc gccctgtcgg 720

gcggggagcg gcggcggctg gaaatcgccc gcgcgctggc cagccagccg cattatatcc 780

tgctggacga accgctggcc ggtatcgacc ccattgcggt gggcgagatc cgtgaccttg 840

tcgcccacct gaaggatcgt ggcatcgggg tgctgattac cgaccacaac gtgcgcgaga 900

cgctggaagt gatcgaccgg gcctacatca tgcacagcgg gcaggtgctg accgagggac 960

ggcccgagga aatcgtggcg aacgaagacg tgcgccgtgt ctatctgggt gaaaaattca 1020

cgctgtaggc ccgtacgcgc gcccctgtgt gacagggtgc atgtgcaagg ggaaggccat 1080

ggaaaaacct gtggtctttt atgcaaggaa ggcatggaca gcaacggtgc aaccatgccc 1140

aaatgtaccc gttcgggacc atgtgcccga caggcacaac gcaaccgcca ccagcacgca 1200

gccagcatgg gagcaggatc gtccccctta tttcatgacc attgactgaa acgaccgtat 1260

<210> 9
<211> 259
<212> PRT
<213> Gluconacetobacter entanii

<400> 9

Met Asn Thr Thr Ser Pro Ala Asp Gln Ala Thr Thr Glu Gln Pro Ile
1               5                   10                  15

Pro Ala Ala Glu Ala Gly Leu Ile Ala Ser Gly Ile Gly Lys Ser Tyr

            20                  25                  30

Lys Lys Arg Gln Val Val Arg Asp Val Ser Leu Gln Val Gln Arg Gly
        35                  40                  45

Glu Ala Val Ala Leu Leu Gly Pro Asn Gly Ala Gly Lys Thr Thr Ser
    50                  55                  60

Phe Tyr Met Ile Val Gly Leu Val Arg Pro Asp Met Gly Thr Ile Thr
65                  70                  75                  80

Leu Asp Gly Ala Asp Ile Thr Gln Leu Pro Met Tyr Arg Arg Ala Arg
                85                  90                  95

Met Gly Ile Gly Tyr Leu Pro Gln Glu Ser Ser Ile Phe Arg Gly Leu
            100                 105                 110

Asn Val Glu Gln Asn Ile Met Ala Ala Leu Glu Ile Val Glu Pro Asp
        115                 120                 125

Arg Asp Arg Arg Gln Thr Met Leu Asp Gly Leu Leu Gly Glu Phe Gly
    130                 135                 140

Ile Thr Arg Leu Arg His Ser Ser Ser Leu Ala Leu Ser Gly Gly Glu
145                 150                 155                 160

Arg Arg Arg Leu Glu Ile Ala Arg Ala Leu Ala Ser Gln Pro His Tyr
                165                 170                 175

Ile Leu Leu Asp Glu Pro Leu Ala Gly Ile Asp Pro Ile Ala Val Gly
            180                 185                 190

Glu Ile Arg Asp Leu Val Ala His Leu Lys Asp Arg Gly Ile Gly Val
        195                 200                 205

Leu Ile Thr Asp His Asn Val Arg Glu Thr Leu Glu Val Ile Asp Arg

210                 215                 220

Ala Tyr Ile Met His Ser Gly Gln Val Leu Thr Glu Gly Arg Pro Glu
225                 230                 235                 240

Glu Ile Val Ala Asn Glu Asp Val Arg Arg Val Tyr Leu Gly Glu Lys
                245                 250                 255

Phe Thr Leu

<210>  10
<211>  30
<212>  DNA
<213>  Artificial

<220>
<223>  Description of Artificial sequence: synthetic oligonucleotide

<400>  10
cgctgacgtc gtgggccgtg ccagaggccc                                        30

<210>  11
<211>  30
<212>  DNA
<213>  Artificial

<220>
<223>  Description of Artificial sequence: synthetic oligonucleotide

<400>  11
ggccaagacg tctgcagcat ggggcgtcac                                        30

<210>  12
<211>  5734
<212>  DNA
<213>  Gluconacetobacter entanii (Acetobacter altoacetigenes MH-24)

<400>  12
catggggcgt cacccccagc ggccagcttg gctacctgat ggacagggcg ggccttctgc      60

aagccctcgg ccactgccat ctgccgggat atgaggccaa atacgaaccg aaggaaaagc     120

gcaccttctg ctaccccacc cagaacgcca gcggctgggc tgtgcagcca tgatcgccaa 180

cccctccctc ttcctgagca attcggaaga gcgatttccg ccgactgaac acgtcgaaaa 240

tggcagtttt ccaccgaaaa aaggaaagga ccataggaaa ggattaatat cttattttta 300

tctaggggtt tgccgatccg cgattttcgc tgggaaaccg ccaaaaatgg cttgccatta 360

ggtcgcacca catgcgacca taaagtcgca cagtgtgcga cctattcggc ccatatacag 420

aggttcccca catgcggaat gtcacccgtc tcaagacccg caaagaccgg ctccgcgagg 480

accaagccga cctgttgaag caagcccttc tgcccttcgc agaggacgat ggaccgatgc 540

gggatgcggt cggacggctc tacgtccaga tcaagaacct caccacccca gaccccggaa 600

ccacggagcc gttcgtcatg atccgtcccg cccagaatcg cgccgtcacc ctctggctgc 660

tgaagaacag taagcggccc atgaaggccg tggacgtatg gacgctgctg ttcgaccacc 720

tgtttcccca taccggccag atcatgctga cccgtgagga aatcgcggaa aaagtcggta 780

tccgggtcaa cgaagttaca gccgtcatga acgagctggt gagcttcggc gcgattttct 840

ccgagcgcga gaaggtggcc ggaatgcgcg ggccgggcct cgcccgctac tacatgaacc 900

ggcatgtggc cgaggtcggc agccgcgcca cgcaggaaga acttgcccta atcccacgcc 960

ccggcgccaa gctggcagtc gtgcagggtg gcaaggctta acccatgaag gtttcggaac 1020

tggacgtgtt cgacagcgcc aaggcggcac aagacccgtt ggtgcgggaa gaactgctgc 1080

aagcagcgca ggcggacggc cacggccccg ccctcgctca tgcccgttcc gtcatagcca 1140

aggcgcgggc cgggcaggac gccaaggctt aacggccccg ccctctcccg cctcgatccc 1200

ggcgggcctg tagcatctcc tgatgctcct tggcgttttt ggcccgctgc tcggcccgct 1260

ctttctcggc cgctgcggct cttaggcgct cttcggccag ccgcatccgc tcgtccatct 1320

gacgtttccg atctgcctcg gcatccttgg cggctcctgc cttcagccct ttgctgaaag 1380

ccatccactt attggcggtt ttctcggctt tctgctgtat cggcggggtc agccggtcaa 1440

atgcctgggc caccctctcg aagccctcac gcatggcgtt gacggcctgc gccagtttag 1500

ccagggcgaa atctatcacc tcggcccgct gggcgttctc ggcccggata cgccggttgt 1560

ggttgccggt cggggtctgg tggcccttcc gttccagagc caccacattc ggccccatgt 1620

gccgctctgg aacgcggtct agcccctgct ccgcattgct ccggtgatct atccgggcct 1680

cttgcccagc ccgctctagc gcggcattgg caaggcccgc ccatagctgc cggatttcct 1740

tcacctcgtc ggcggccttc cccagtccca tgccctgccg cttcttgtcg gacagttcga 1800

tggttgattt gtctccaaag gacagcttgc catcggcccc ccgctccacc gtgcgggtgg 1860

tggtcatgat gtgcgcgtga tgattccggt cgtcgccctc gtcacccgga agatgcacgg 1920

ccacgtccac ggccaccccg taccgctgga ccaactcacg cgcgaaactg tccgccagtt 1980

cggcccgctg ctcgctggtg agttcatgag ggagggccac aacccattcc ctcccggtgc 2040

gggcgtcctt gcgtttctct gatcgctccg cgtcattcca caattccgaa cggtcagcgg 2100

tgccaccccc cggaatgaaa attgccttat gggcaacgct attctgcctg gggctgtatt 2160

tgtgttcgtg cccgtcaacc tcgttggtca aatcctcgcc agcacgatac gcagccgcag 2220

ccacaacgga acgccctgcg ctccggctga tcggtttcgt ttctgcgcga tagattgcca 2280

cggatcgagc gcctaccttt tggagttaaa cggggggttc aggggggcga agccaccatg 2340

acgcaggact tgcacttgtg caagtcgtaa ctgcgccctt aatacctgac ggcatcaagg 2400

gatatgtggt attcgtttga aacggaacgg ctccacggtg aggatgatat gagcgatatt 2460

gcgaaagaga ttgagaacgc caaaaggatc atagctgaac agaaaaagcg catcaaagat 2520

gcccagaagg aagcagctaa agcggaatca aagttgaggg accgtcagaa ctacatcttg 2580

ggcggcgcac tggtaaaact tgccgaaaca gatgaacggg ccgtccgcac tattgaaaca 2640

cttttgaagc tggtggatcg tccatcagac cggaaggcgt ttgaggtgtt ttcccgtctc 2700

ccatccctct ccctgcccac gcagccagca ccggacaccg gccatgagtg aggcactgga 2760

agaagatccg tttgaactgt tcaaaagggt cgaaaaaagc ctgtccacgg ccaccgccag 2820

catggagcgg ctggccgccg aacaagatgc caggtgcaag accatttcag acgccgccgg 2880

aaaagcctct aaattggccg aggaagccgg tgacaccttc acagcatcca agaggcgtct 2940

gatgatctgg acggccctct gcgcggctct gctggtctgt ggcgggtggt tggcgggtta 3000

ttggctggga caccgtgacg gttgggcctc tggcacggcc cacgacgtct aagaaaccat 3060

tattatcatg acattaacct ataaaaatag gcgtatcacg aggccctttc gtctcgcgcg 3120

tttcggtgat gacggtgaaa acctctgaca catgcagctc ccggagacgg tcacagcttg 3180

tctgtaagcg gatgccggga gcagacaagc ccgtcagggc gcgtcagcgg gtgttggcgg 3240

gtgtcggggc tggcttaact atgcggcatc agagcagatt gtactgagag tgcaccatat 3300

gcggtgtgaa ataccgcaca gatgcgtaag gagaaaatac cgcatcaggc gccattcgcc 3360

attcaggctg cgcaactgtt gggaagggcg atcggtgcgg gcctcttcgc tattacgcca 3420

gctggcgaaa gggggatgtg ctgcaaggcg attaagttgg gtaacgccag ggttttccca 3480

gtcacgacgt tgtaaaacga cggccagtgc caagcttgca tgcctgcagg tcgactctag 3540

aggatccccg ggtaccgagc tcgaattcgt aatcatggtc atagctgttt cctgtgtgaa 3600

attgttatcc gctcacaatt ccacacaaca tacgagccgg aagcataaag tgtaaagcct 3660

ggggtgccta atgagtgagc taactcacat taattgcgtt gcgctcactg cccgctttcc 3720

agtcgggaaa cctgtcgtgc cagctgcatt aatgaatcgg ccaacgcgcg gggagaggcg 3780

gtttgcgtat tgggcgctct tccgcttcct cgctcactga ctcgctgcgc tcggtcgttc 3840

ggctgcggcg agcggtatca gctcactcaa aggcggtaat acggttatcc acagaatcag 3900

gggataacgc aggaaagaac atgtgagcaa aaggccagca aaaggccagg aaccgtaaaa 3960

aggccgcgtt gctggcgttt ttccataggc tccgcccccc tgacgagcat cacaaaaatc 4020

gacgctcaag tcagaggtgg cgaaacccga caggactata aagataccag gcgtttcccc 4080

ctggaagctc cctcgtgcgc tctcctgttc cgaccctgcc gcttaccgga tacctgtccg 4140

cctttctccc ttcgggaagc gtggcgcttt ctcaatgctc acgctgtagg tatctcagtt 4200

cggtgtaggt cgttcgctcc aagctgggct gtgtgcacga accccccgtt cagcccgacc 4260

gctgcgcctt atccggtaac tatcgtcttg agtccaaccc ggtaagacac gacttatcgc 4320

cactggcagc agccactggt aacaggatta gcagagcgag gtatgtaggc ggtgctacag 4380

agttcttgaa gtggtggcct aactacggct acactagaag gacagtattt ggtatctgcg 4440

ctctgctgaa gccagttacc ttcggaaaaa gagttggtag ctcttgatcc ggcaaacaaa 4500

ccaccgctgg tagcggtggt ttttttgttt gcaagcagca gattacgcgc agaaaaaaag 4560

gatctcaaga agatcctttg atcttttcta cggggtctga cgctcagtgg aacgaaaact 4620

cacgttaagg gattttggtc atgagattat caaaaaggat cttcacctag atccttttaa 4680

attaaaaatg aagttttaaa tcaatctaaa gtatatatga gtaaacttgg tctgacagtt 4740

accaatgctt aatcagtgag gcacctatct cagcgatctg tctatttcgt tcatccatag 4800

ttgcctgact ccccgtcgtg tagataacta cgatacggga gggcttacca tctggcccca 4860

gtgctgcaat gataccgcga gacccacgct caccggctcc agatttatca gcaataaacc 4920

agccagccgg aagggccgag cgcagaagtg gtcctgcaac tttatccgcc tccatccagt 4980

ctattaattg ttgccgggaa gctagagtaa gtagttcgcc agttaatagt ttgcgcaacg 5040

ttgttgccat tgctacaggc atcgtggtgt cacgctcgtc gtttggtatg gcttcattca 5100

gctccggttc ccaacgatca aggcgagtta catgatcccc catgttgtgc aaaaaagcgg 5160

ttagctcctt cggtcctccg atcgttgtca gaagtaagtt ggccgcagtg ttatcactca 5220

tggttatggc agcactgcat aattctctta ctgtcatgcc atccgtaaga tgcttttctg 5280

tgactggtga gtactcaacc aagtcattct gagaatagtg tatgcggcga ccgagttgct 5340

cttgcccggc gtcaatacgg gataataccg cgccacatag cagaacttta aaagtgctca 5400

tcattggaaa acgttcttcg gggcgaaaac tctcaaggat cttaccgctg ttgagatcca 5460

ttcgatgtaa cccactcgtg cacccaactg atcttcagca tcttttactt tcaccagcgt 5520

ttctgggtga gcaaaaacag gaaggcaaaa tgccgcaaaa aagggaataa gggcgacacg 5580

gaaatgttga atactcatac tcttcctttt tcaatattat tgaagcattt atcagggtta 5640

ttgtctcatg agcggataca tatttgaatg tatttagaaa aataaacaaa taggggttcc 5700

gcgcacattt ccccgaaaag tgccacctga cgtc 5734

<210> 13
<211> 30

<212>  DNA
<213>  Artificial

<220>
<223>  Description of Artificial Sequence: synthetic oligonucleotide

<400>  13
tgaacatcct tgaattcgcg aagcggcgtt                                        30


<210>  14
<211>  27
<212>  DNA
<213>  Artificial

<220>
<223>  Description of Artificial Sequence: synthetic oligonucleotide

<400>  14
acggatccag gcgtccggcc tgatcat                                           27


<210>  15
<211>  22
<212>  DNA
<213>  Artificial

<220>
<223>  Description of Artificial Sequence: synthetic oligonucleotide

<400>  15
aagcacgaga tcgagtggaa at                                                22


<210>  16
<211>  22
<212>  DNA
<213>  Artificial

<220>
<223>  Description of Artificial Sequence: synthetic oligonucleotide

<400>  16
gggtgtcagg tcaagaatga tg                                                22


<210>  17
<211>  20

<212>  DNA
<213>  Artificial

<220>
<223>  Description of Artificial sequence: synthetic oligonucleotide

<400>  17
gccatcgtga acatgcatac                                                    20


<210>  18
<211>  22
<212>  DNA
<213>  Artificial

<220>
<223>  Description of Artificial sequence: synthetic oligonucleotide

<400>  18
ccttccttgc ataaaagacc ac                                                 22